# EUROPEAN PATENT APPLICATION

(11) **EP 4 445 913 A2**
(43) Date of publication of application: **16.10.2024**
(21) Application number: 24174873.0
(22) Date of filing: 01.08.2019
(51) Int. Cl.: A61K 39/395

(54) **A PHARMACEUTICAL COMPOSITION FOR USE IN THE TREATMENT OR PREVENTION OF A C5-RELATED DISEASE AND A METHOD FOR TREATING OR PREVENTING A C5-RELATED DISEASE**

(30) Priority: 01.08.2018 US 201862713211 P; 17.08.2018 US 201862764952 P; 13.11.2018 US 201862760204 P
(62) Divisional of application: 19755447.0
(71) Applicant: Chugai Seiyaku Kabushiki Kaisha, Kita-ku Tokyo 115-8543 (JP); Osaka University, Suita-shi, Osaka 565-0871 (JP); F. Hoffmann-La Roche AG, 4070 Basel (CH)
(72) Inventor: SHINOMIYA, Kenji, Tokyo, 103-8324 (JP); GOTANDA, Keisuke, Tokyo, 103-8324 (JP); NISHIMURA, Jun-ichi, Suita-shi, Osaka, 565-0871 (JP); WINTER, Erica, New York, 10016 (US); HSU, Joy C., New York, 10016 (US)
(74) Representative: Vossius & Partner Patentanwälte Rechtsanwälte mbB

(57) **Abstract**

The present invention relates to pharmaceutical compositions for use in the treatment or prevention of a C5-related disease and methods for treating or preventing a C5-related disease. The present invention further relates to dosages and administrations of anti-C5 antibody or pharmaceutical compositions containing the anti-C5 antibody.

## Description

### Technical Field

The present invention relates to dosages and administrations of anti-C5 antibody.

### Background Art

The complement system plays a central role in the clearance of immune complexes and in immune responses to infectious agents, foreign antigens, virus-infected cells and tumor cells. There are about 25-30 complement proteins, which are found as a complex collection of plasma proteins and membrane cofactors. Complement components achieve their immune defensive functions by interacting in a series of intricate enzymatic cleavages and membrane binding events. The resulting complement cascades lead to the production of products with opsonic, immunoregulatory, and lytic functions.

Currently, it is widely accepted that the complement system can be activated through three distinct pathways: the classical pathway, the lectin pathway, and the alternative pathway. These pathways share many components, and while they differ in their initial steps, they converge and share the same terminal complement components (C5 through C9) responsible for the activation and destruction of target cells.

The classical pathway is normally activated by the formation of antigen-antibody complexes. Independently, the first step in activation of the lectin pathway is the binding of specific lectins such as mannan-binding lectin (MBL), H-ficolin, M-ficolin, L-ficolin and C-type lectin CL-11. In contrast, the alternative pathway spontaneously undergoes a low level of turnover activation, which can be readily amplified on foreign or other abnormal surfaces (bacteria, yeast, virally infected cells, or damaged tissue). These pathways converge at a point where complement component C3 is cleaved by an active protease to yield C3a and C3b.

C3a is an anaphylatoxin. C3b binds to bacterial and other cells, as well as to certain viruses and immune complexes, and tags them for removal from the circulation (the role known as opsonin). C3b also forms a complex with other components to form C5 convertase, which cleaves C5 into C5a and C5b.

C5 is a 190 kDa protein found in normal serum at approximately 80 micro g/ml (0.4 micro M). C5 is glycosylated with about 1.5-3% of its mass attributed to carbohydrate. Mature C5 is a heterodimer of 115 kDa alpha chain that is disulfide linked to 75 kDa beta chain. C5 is synthesized as a single chain precursor protein (pro-C5 precursor) of 1676 amino acids (see, e.g., PTL1 and PTL2). The pro-C5 precursor is cleaved to yield the beta chain as an amino terminal fragment and the alpha chain as a carboxyl terminal fragment. The alpha chain and the beta chain polypeptide fragments are connected to each other via a disulfide bond and constitute the mature C5 protein.

Mature C5 is cleaved into the C5a and C5b fragments during activation of the complement pathways. C5a is cleaved from the alpha chain of C5 by C5 convertase as an amino terminal fragment comprising the first 74 amino acids of the alpha chain. The remaining portion of mature C5 is fragment C5b, which contains the rest of the alpha chain disulfide bonded to the beta chain. Approximately 20% of the 11 kDa mass of C5a is attributed to carbohydrate.

C5a is another anaphylatoxin. C5b combines with C6, C7, C8 and C9 to form the membrane attack complex (MAC, C5b-9, terminal complement complex (TCC)) at the surface of the target cell. When sufficient numbers of MACs are inserted into target cell membranes, MAC pores are formed to mediate rapid osmotic lysis of the target cells.

As mentioned above, C3a and C5a are anaphylatoxins. They can trigger mast cell de-granulation, which releases histamine and other mediators of inflammation, resulting in smooth muscle contraction, increased vascular permeability, leukocyte activation, and other inflammatory phenomena including cellular proliferation resulting in hypercellularity. C5a also functions as a chemotactic peptide that serves to attract granulocytes such as neutrophils, eosinophils, basophils and monocytes to the site of complement activation.

The activity of C5a is regulated by the plasma enzyme carboxypeptidase N that removes the carboxy-terminal arginine from C5a forming C5a-des-Arg derivative. C5a-des-Arg exhibits only 1% of the anaphylactic activity and polymorphonuclear chemotactic activity of unmodified C5a.

While a properly functioning complement system provides a robust defense against infecting microbes, inappropriate regulation or activation of complement has been implicated in the pathogenesis of a variety of disorders including, e.g., rheumatoid arthritis (RA); lupus nephritis; ischemia-reperfusion injury; paroxysmal nocturnal hemoglobinuria (PNH); atypical hemolytic uremic syndrome (aHUS); dense deposit disease (DDD); macular degeneration (e.g., age-related macular degeneration (AMD)); hemolysis, elevated liver enzymes, and low platelets (HELLP) syndrome; thrombotic thrombocytopenic purpura (TTP); spontaneous fetal loss; Pauci-immune vasculitis; epidermolysis bullosa; recurrent fetal loss; multiple sclerosis (MS); traumatic brain injury; and injury resulting from myocardial infarction, cardiopulmonary bypass and hemodialysis (see, e.g., NPL1). Therefore, inhibition of excessive or uncontrolled activations of the complement cascade can provide clinical benefits to patients with such disorders.

Paroxysmal nocturnal hemoglobinuria (PNH) is an uncommon blood disorder, wherein red blood cells are compromised and are thus destroyed more rapidly than normal red blood cells. PNH results from the clonal expansion of hematopoietic stem cells with somatic mutations in the PIG-A (phosphatidylinositol glycan class A) gene which is located on the X chromosome. Mutations in PIG-A lead to an early block in the synthesis of glycosylphosphatidylinositol (GPI), a molecule which is required for the anchor of many proteins to cell surfaces. Consequently, PNH blood cells are deficient in GPI-anchored proteins, which include complement-regulatory proteins CD55 and CD59. Under normal circumstances, these complement-regulatory proteins block the formation of MAC on cell surfaces, thereby preventing erythrocyte lysis. The absence of the GPI-anchored proteins causes complement-mediated hemolysis in PNH.

PNH is characterized by hemolytic anemia (a decreased number of red blood cells), hemoglobinuria (the presence of hemoglobin in urine, particularly evident after sleeping), and hemoglobinemia (the presence of hemoglobin in the bloodstream). PNH-afflicted individuals are known to have paroxysms, which are defined here as incidences of dark-colored urine. Hemolytic anemia is due to intravascular destruction of red blood cells by complement components. Other known symptoms include dysphasia, fatigue, erectile dysfunction, thrombosis and recurrent abdominal pain.

Eculizumab is a humanized monoclonal antibody directed against the complement protein C5, and the first therapy approved for the treatment of paroxysmal nocturnal hemoglobinuria (PNH) and atypical hemolytic uremic syndrome (aHUS) (see, e.g., NPL2). Eculizumab inhibits the cleavage of C5 into C5a and C5b by C5 convertase, which prevents the generation of the terminal complement complex C5b-9. Both C5a and C5b-9 cause the terminal complement-mediated events that are characteristic of PNH and aHUS (see also, PTL3, PTL4, PTL5, and PTL6).

Several reports have described anti-C5 antibodies. For example, PTL7 described an anti-C5 antibody which binds to the alpha chain of C5 but does not bind to C5a, and blocks the activation of C5, while PTL8 described an anti-C5 monoclonal antibody which inhibits C5a formation. On the other hand, PTL9 described an anti-C5 antibody which recognizes the proteolytic site for C5 convertase on the alpha chain of C5, and inhibits the conversion of C5 to C5a and C5b. PTL10 described an anti-C5 antibody which has an affinity constant of at least 1 ×10⁷ M⁻¹.

Antibodies (IgGs) bind to neonatal Fc receptor (FcRn), and have long plasma retention times. The binding of IgGs to FcRn is typically observed under acidic conditions (e.g., pH 6.0), and it is rarely observed under neutral conditions (e.g., pH 7.4). Typically, IgGs are nonspecifically incorporated into cells via endocytosis, and return to the cell surfaces by binding to endosomal FcRn under the acidic conditions in the endosomes. Then, IgGs dissociate from FcRn under the neutral conditions in plasma. IgGs that have failed to bind to FcRn are degraded in lysosomes. When the FcRn binding ability of an IgG under acidic conditions is eliminated by introducing mutations into its Fc region, the IgG is not recycled from the endosomes into the plasma, leading to marked impairment of the plasma retention of the IgG. To improve the plasma retention of IgGs, a method that enhances their FcRn binding under acidic conditions has been reported. When the FcRn binding of an IgG under acidic conditions is improved by introducing an amino acid substitution into its Fc region, the IgG is more efficiently recycled from the endosomes to the plasma, and thereby shows improved plasma retention. Meanwhile, it has also been reported that an IgG with enhanced FcRn binding under neutral conditions does not dissociate from FcRn under the neutral conditions in plasma even when it returns to the cell surface via its binding to FcRn under the acidic conditions in the endosomes, and consequently its plasma retention remains unaltered, or rather, is worsened (see, e.g., NPL3; NPL4; NPL5).

Recently, antibodies that bind to antigens in a pH-dependent manner have been reported (see, e.g., PTL11 and PTL12). These antibodies strongly bind to antigens under the plasma neutral conditions and dissociate from the antigens under the endosomal acidic conditions. After dissociating from the antigens, the antibodies become capable once again of binding to antigens when recycled to the plasma via FcRn. Thus, a single antibody molecule can repeatedly bind to multiple antigen molecules. In general, the plasma retention of an antigen is much shorter than that of an antibody that has the above-mentioned FcRn-mediated recycling mechanism. Therefore, when an antigen is bound to an antibody, the antigen normally shows prolonged plasma retention, resulting in an increase of the plasma concentration of the antigen. On the other hand, it has been reported that the above-described antibodies, which bind to antigens in a pH-dependent manner, eliminate antigens from plasma more rapidly than typical antibodies because they dissociate from the antigens within the endosomes during the FcRn-mediated recycling process. PTL13 also described computer modeling analysis showing that an antibody with pH-dependent binding directed against C5 could extend antigen knockdown.

### Citation List

### Patent Literature

[PTL1] US Patent No. 6,355,245
[PTL2] US Patent No. 7,432,356
[PTL3] WO 2005/074607
[PTL4] WO 2007/106585
[PTL5] WO 2008/069889
[PTL6] WO 2010/054403
[PTL7] WO 95/29697
[PTL8] WO 2002/30985
[PTL9] WO 2004/007553
[PTL10] WO 2010/015608
[PTL11] WO 2009/125825
[PTL12] WO 2011/122011
[PTL13] WO 2011/111007

### Non Patent Literature

[NPL1] Holers et al., Immunol. Rev. 223:300-316 (2008)
[NPL2] Dmytrijuk et al., The Oncologist 13(9):993-1000 (2008)
[NPL3] Yeung et al., J Immunol. 182(12): 7663-7671 (2009)
[NPL4] Datta-Mannan et al., J Biol. Chem. 282(3):1709-1717 (2007)
[NPL5] Dall'Acqua et al., J. Immunol. 169(9):5171-5180 (2002)

### Summary of Invention

The invention provides pharmaceutical compositions for use in the treatment or prevention of a C5-related disease and methods for treating or preventing a C5-related disease. The invention also provides dosages and administrations of anti-C5 antibody or pharmaceutical compositions containing the anti-C5 antibody.

The inventors of the invention investigated more effective and safer dosages and administrations of anti-C5 antibody for use in the treatment and/or prevention of C5-related diseases, which can also reduce burden on patients. From such standpoints, the inventors found that administration in a lower dose at least once before administration in high doses at long intervals is favorable for the treatment or prevention of a C5-related disease by subcutaneous admininstration of anti-C5 antibody.

The inventors also discovered suitable conditions for dosing, including a dose of the antibody, frequency and intervals of the subcutaneous administrations.

Specifically, the present invention relates to:
[1] A pharmaceutical composition for use in a method of treatment or prevention of a C5-related disease, which is formulated for subcutaneous injection, and comprises an anti-C5 antibody, wherein the composition is subcutaneously administered in two phases,
   wherein in both phases there is an interval between every two subcutaneous administrations, wherein each phase comprises at least one interval, and wherein in the first phase
   i) the at least one interval is shorter than the at least one interval in the second phase, and
   ii) the dose of the antibody per administration is lower than or the same as the antibody dose per administration in the second phase;
[2] The pharmaceutical composition for use of [1], wherein the at least one interval in the first phase is 1 day to 2 months;
[3] The pharmaceutical composition for use of [1] or [2], wherein the at least one interval in the first phase is 5 days to 14 days;
[4] The pharmaceutical composition for use of any one of [1] to [3], wherein the at least one interval in the second phase is 2 days to 6 months;
[5] The pharmaceutical composition for use of any one of [1] to [4], wherein said at least one interval in the second phase is 15 days to 3 months;
[6] The pharmaceutical composition for use of any one of [1] to [5], wherein the dose of the antibody in the subcutaneous administration of the first phase is 50 mg to 350 mg;
[7] The pharmaceutical composition for use of any one of [1] to [6], wherein the dose of the antibody of the subcutaneous administration of the first phase is 150 mg to 200 mg and lower than the dose of subcutaneous administration of the second phase, preferably wherein the antibody dose in the first phase is 170mg;
[8] The pharmaceutical composition for use of any one of [1] to [6], wherein the dose of the antibody of the subcutaneous administration of the first phase is 300 mg to 350 mg and the same as the antibody dose of the subcutaneous administration of the second phase;
[9] The pharmaceutical composition for use of any one of [1] to [8], wherein number of subcutaneous administrations in the first phase is 1 to 12;
[10] The pharmaceutical composition for use of any one of [1] to [9], number of subcutaneous administrations in the first phase is 5 to 10, preferably wherein the number is 8;
[11] The pharmaceutical composition for use of any one of [1] to [10], wherein the dose of the antibody per administration in the second phase is 350 mg to 1,000 mg, or 650 mg to 700 mg;
[12] The pharmaceutical composition for use of any one of [1] to [7] and [9] to [11], wherein the anti-C5 antibody dose per administration in the first phase is three to five folds lower than the anti-C5 antibody dose per administration in the second phase;
[13] The pharmaceutical composition for use of any one of [1] to [12], wherein a pharmaceutical composition formulated for intravenous administration and comprising an anti-C5 antibody is intravenously administrated before the first subcutaneous administration of the first phase;
[14] The pharmaceutical composition for use of [13], wherein the first subcutaneous administration of the first phase is administrated 0 days to 1 month after the final administration of the intravenously administrated pharmaceutical composition;
[15] The pharmaceutical composition for use of [13] or [14], wherein the antibody dose of said intravenous administration is 100 to 2,000 mg;
[16] The pharmaceutical composition for use of any one of [1] to [15], wherein the C5-related disease is any one selected from a group consisting of rheumatoid arthritis (RA); lupus nephritis; ischemia-reperfusion injury; paroxysmal nocturnal hemoglobinuria (PNH); atypical hemolytic uremic syndrome (aHUS); dense deposit disease (DDD); macular degeneration; hemolysis, elevated liver enzymes, and low platelets (HELLP) syndrome; thrombotic thrombocytopenic purpura (TTP); spontaneous fetal loss; Pauci-immune vasculitis; epidermolysis bullosa; recurrent fetal loss; multiple sclerosis (MS); traumatic brain injury; and injury resulting from myocardial infarction, cardiopulmonary bypass or hemodialysis;
[17] A method for treating or preventing a C5-related disease, wherein the method comprises subcutaneously administering to a subject a pharmaceutical composition, which is formulated for subcutaneous injection and comprises an anti-C5 antibody, wherein the composition is subcutaneously administered in two phases,
   wherein in both phases there is an interval between every two subcutaneous administrations, wherein each phase comprises at least one interval, and wherein in the first phase
   i) the at least one interval is shorter than the at least one interval in the second phase, and
   ii) the dose of the antibody per administration is lower than or the same as the antibody dose per administration in the second phase;
[18] Use of an anti-C5 antibody in the manufacture of a pharmaceutical composition for treating or preventing a C5-related disease, wherein the composition is formulated for subcutaneous injection, comprises the anti-C5 antibody, and wherein the composition is subcutaneously administered in two phases,
   wherein in both phases there is an interval between every two subcutaneous administrations, wherein each phase comprises at least one interval, and wherein in the first phase
   i) the at least one interval is shorter than the at least one interval in the second phase, and
   ii) the dose of the antibody per administration is lower than or the same as the antibody dose per administration in the second phase; and
[19] A product for treating or preventing a C5-related disease, comprising (a) a container; (b) a pharmaceutical composition in the container, wherein the pharmaceutical composition is formulated for subcutaneous injection and comprises an anti-C5 antibody; and (c) a document instructing that the pharmaceutical composition is subcutaneously administered in two phases,
   wherein in both phases there is an interval between every two subcutaneous administrations, wherein each phase comprises at least one interval, and wherein in the first phase
   i) the at least one interval is shorter than the at least one interval in the second phase, and
   ii) the dose of the antibody per administration is lower than or the same as the antibody dose per administration in the second phase.

### Brief Description of Drawings

[fig.1] Panels A and B in Fig. 1 show the change in plasma 305LO15 concentration in cynomolgus monkeys over time by subcutaneous injection (A) or intravenous injection (B) of antibody 305LO15. The plasma 305LO15 concentration was measured by ELISA. Each point shows a mean value from three males or three females in (A) and a mean value from six males or six females in (B).
[fig.2]Fig. 2 shows the change in plasma 305LO15 concentration in cynomolgus monkeys over time to which monkeys an initial intravenous injection and subsequent subcutaneous maintenance injections of antibody 305LO15 were administered. The plasma 305LO15 concentration was measured by ELISA. Each point shows a mean value from five males or five females.
[fig.3A]Fig. 3A is a graph in which the plasma concentrations of free C5 are plotted against each plasma concentration of the antibody for each indicated dosing amount and route. IV, intravenous injection; SC, subcutaneous injection.
[fig.3B]Fig. 3B is a graph in which the measured complement activities are plotted against each plasma concentration of the antibody for each indicated dosing amount and route. IV, intravenous injection; SC, subcutaneous injection.
[fig.4A]Fig. 4A shows the simulated time-courses of plasma 305LO15 concentration in dosing regimens designed for Part 1 clinical study. IV, intravenous injection; SC, subcutaneous injection.
[fig.4B]Fig. 4B shows the simulated time-course of plasma 305LO15 concentration in dosing regimen designed for Part 2 clinical study. SC, subcutaneous injection; QW, once-every-week administration.
[fig.4C]Fig. 4C shows the simulated time-courses of plasma 305LO15 concentration in dosing regimens designed for Part 3 clinical study. SC, subcutaneous injection; QW, once-every-week administration; Q2W, once-every-two-week administration; Q4W, once-every-four-week administration.
[fig.5]Fig. 5 shows the profile of immunocomplexes formed with eculizumab (ECZ), human C5 (hC5), and/or 305LO15 analyzed by in vitro size-exclusion chromatography at pH7.4 (top) and at pH6.0 (bottom).
[fig.6]Fig. 6 shows the individual observed and simulated plasma 305LO15 concentration-time profiles in healthy subjects who received 75 mg IV infusion (over 60 min). The gray shaded area is the 95% prediction interval of the simulated 305LO15 concentration-time profile. The solid line indicated by an arrow is the simulated median 305LO15 concentration-time profile. The dashed line denotes the 40 micro g/ mL PD threshold.
[fig.7]Fig. 7 shows the individual observed and simulated plasma 305EO15 concentration-time profiles in healthy subjects who received 125 mg IV infusion (over 60 min). The gray shaded area is the 95% prediction interval of the simulated 305LO15 concentration-time profile. The solid line indicated by an arrow is the simulated median 305LO15 concentration-time profile. The dashed line denotes the 40 micro g/ mL PD threshold.
[fig.8]Fig. 8 shows the individual observed and simulated plasma 305EO15 concentration-time profiles in healthy subjects who received 100 mg SC. The gray shaded area is a 95% prediction interval of the simulated 305LO15 concentration-time profile. The solid line indicated by an arrow is the simulated median 305LO15 concentration-time profile. For simulations conducted for the single 100 mg SC dosing, a bioavailability of 90% was expected. The dashed line denotes the 40 micro g/mL PD threshold.
[fig.9]Fig. 9 shows the relationship between plasma 305LO15 concentrations and hemolytic activity (LIA) following a single IV infusion or SC injection to healthy subjects. Ex vivo LIA dose-response curve was generated using human plasma samples spiked with various concentrations of 305LO15.
[fig.10A]Fig. 10A shows the time profile of serum LDH levels in a PNH patient, patient X, who received an IV dose of 375 mg 305LO15 on day 1, an IV dose of 500 mg 305LO15 on day 8, an IV dose of 1000 mg 305LO15 on day 22, an SC dose of 170 mg 305LO15 on day 36, and an SC dose of 170 mg 305LO15 on day 43 in the Part 2 study.
[fig.10B] Fig. 10B shows the time profile of hemolytic activity (LIA) in the PNH patient, patient X, who received an IV dose of 375 mg 305LO15 on day 1, an IV dose of 500 mg 305LO15 on day 8, an IV dose of 1000 mg 305LO15 on day 22, an SC dose of 170 mg 305LO15 on day 36, and an SC dose of 170 mg 305LO15 on day 43 in the Part 2 study.
[fig.11] Fig. 11 shows the optimised SC dose-regimen in Part 3 of Study BP39144.

### Description of Embodiments

The techniques and procedures described or referenced herein are generally well understood and commonly employed using conventional methodology by those skilled in the art, such as, for example, the widely utilized methodologies described in Sambrook et al., Molecular Cloning: A Laboratory Manual 3d edition (2001) Cold Spring Harbor Laboratory Press, Cold Spring Harbor, N.Y.; Current Protocols in Molecular Biology (F.M. Ausubel, et al. eds., (2003)); the series Methods in Enzymology (Academic Press, Inc.): PCR 2: A Practical Approach (M.J. MacPherson, B.D. Hames and G.R. Taylor eds. (1995)), Harlow and Lane, eds. (1988) Antibodies, A Laboratory Manual, and Animal Cell Culture (R.I. Freshney, ed. (1987)); Oligonucleotide Synthesis (M.J. Gait, ed., 1984); Methods in Molecular Biology, Humana Press; Cell Biology: A Laboratory Notebook (J.E. Cellis, ed., 1998) Academic Press; Animal Cell Culture (R.I. Freshney), ed., 1987); Introduction to Cell and Tissue Culture (J. P. Mather and P.E. Roberts, 1998) Plenum Press; Cell and Tissue Culture: Laboratory Procedures (A. Doyle, J.B. Griffiths, and D.G. Newell, eds., 1993-8) J. Wiley and Sons; Handbook of Experimental Immunology (D.M. Weir and C.C. Blackwell, eds.); Gene Transfer Vectors for Mammalian Cells (J.M. Miller and M.P. Calos, eds., 1987); PCR: The Polymerase Chain Reaction, (Mullis et al., eds., 1994); Current Protocols in Immunology (J.E. Coligan et al., eds., 1991); Short Protocols in Molecular Biology (Wiley and Sons, 1999); Immunobiology (C.A. Janeway and P. Travers, 1997); Antibodies (P. Finch, 1997); Antibodies: A Practical Approach (D. Catty., ed., IRL Press, 1988-1989); Monoclonal Antibodies: A Practical Approach (P. Shepherd and C. Dean, eds., Oxford University Press, 2000); Using Antibodies: A Laboratory Manual (E. Harlow and D. Lane (Cold Spring Harbor Laboratory Press, 1999); The Antibodies (M. Zanetti and J. D. Capra, eds., Harwood Academic Publishers, 1995); and Cancer: Principles and Practice of Oncology (V.T. DeVita et al., eds., J.B. Lippincott Company, 1993).

### I. Definitions

"Interval" (an interval between individual administrations) indicates an interval between administration of the n^{th} dose (n is an integer of 1 or greater) and administration of the (n+1)^{th} dose.

### II. A pharmaceutical composition for subcutaneous injection

A pharmaceutical composition for use in a method of treatment or prevention of a C5-related disease is provided. The pharmaceutical composition is formulated for subcutaneous injection, and comprises an anti-C5 antibody, wherein the composition is subcutaneously administered in two phases, wherein in both phases there is an interval between every two subcutaneous administrations, wherein each phase comprises at least one interval, and wherein in the first phase
i) the at least one interval is shorter than the at least one interval in the second phase, and
ii) the dose of the antibody per administration is lower than or the same as the antibody dose per administration in the second phase.

The subcutaneous injection can be carried out using ordinary devices and methods to administer a pharmaceutical composition to subcutaneous tissue by injection. Specific devices and methods for the subcutaneous injection also may be selected.

### Exemplary Anti-CS Antibodies

The anti-C5 antibody is not limited to a specific embodiment and can be suitably selected from antibodies known as anti-C5 antibody. The term "anti-C5 antibody", or "an antibody that binds to C5" refers to an antibody that is capable of binding C5 with sufficient affinity such that the antibody is useful as a therapeutic agent in targeting C5.

In one aspect, the anti-C5 antibodies inhibit activation of C5. In certain embodiments, anti-C5 antibodies prevent the cleavage of C5 to form C5a and C5b, thus preventing the generation of anaphylatoxic activity associated with C5a, as well as preventing the assembly of the C5b-9 membrane attack complex (MAC) associated with C5b. In certain embodiments, anti-C5 antibodies block the conversion of C5 into C5a and C5b by C5 convertase. In certain embodiments, anti-C5 antibodies block access of the C5 convertase to the cleavage site on C5. In certain embodiments, anti-C5 antibodies block hemolytic activity caused by the activation of C5. In further embodiments, anti-C5 antibodies inhibit the activation of C5 via classical pathway and/or alternative pathway.

In one aspect, the pharmaceutical compositions of the present invention are useful in treating or preventing a C5-related disease at least in part based on the above-mentioned activities of the anti-C5 antibodies in inhibiting activation of C5.

In certain embodiments, C5 activity can be measured as a function of its cell-lysing ability in a subject's body fluids. The cell-lysing ability, or a reduction thereof, of C5 can be measured by methods well known in the art, for example, a conventional hemolytic assay, such as the hemolysis assay described by Kabat and Mayer (eds), Experimental Immunochemistry, 2nd Edition, 135-240, Springfield, IL, CC Thomas (1961), pages 135-139, or a conventional variation of that assay, such as the chicken erythrocyte hemolysis method as described in, e.g., Hillmen et al., N. Engl. J. Med. 350(6): 552-559 (2004). In certain embodiments, C5 activity, or inhibition thereof, is quantified using a CH50eq assay. The CH50eq assay is a method for measuring the total classical complement activity in serum. This test is a lytic assay, which uses antibody-sensitized erythrocytes as the activator of the classical complement pathway, and various dilutions of the test serum to determine the amount required to give 50% lysis (CH50). The percentage of hemolysis can be determined, for example, using a spectrophotometer. The CH50eq assay provides an indirect measure of terminal complement complex (TCC) formation, since the TCC themselves are directly responsible for the hemolysis measured. Inhibition of C5 activation can also be detected and/or measured using the methods set forth and exemplified in the working examples. Using assays of these or other suitable types, candidate antibodies capable of inhibiting the activation of C5 can be screened. In certain embodiments, inhibition of C5 activation includes at least a 5%, 10%, 15%, 20%, 25%, 30%, 35%, or 40% or greater decrease in the C5 activation in an assay as compared to the effect of a negative control under similar conditions. In some embodiments, it refers to inhibition of C5 activation by at least 45%, 50%, 55%, 60%, 65%, 70%, 75%, 80%, 85%, 90%, or 95% or greater.

In preferred embodiments, an epitope of the anti-C5 antibody is different from the epitope of eculizumab.

In certain embodiments, the anti-C5 antibody for the present invention binds to an epitope within the beta chain of C5. In certain embodiments, the anti-C5 antibody binds to an epitope within the MG1-MG2 domain of the beta chain of C5. In certain embodiments, the anti-C5 antibody binds to an epitope within a fragment consisting of amino acids 19-180 of the beta chain of C5. In certain embodiments, the anti-C5 antibody binds to an epitope within the MG1 domain (amino acids 20-124 of SEQ ID NO: 1) of the beta chain of C5. In certain embodiments, the anti-C5 antibody binds to an epitope within a fragment consisting of amino acids 33-124 of the beta chain of C5 (SEQ ID NO: 1).

In certain embodiments, an anti-C5 antibody for the present invention binds to an epitope within the beta chain of C5 which consists of the MG1 domain. In certain embodiments, an anti-C5 antibody binds to an epitope within the beta chain (SEQ ID NO: 1) of C5 which comprises at least one fragment selected from the group consisting of amino acids 47-57, 70-76, and 107-110. In certain embodiments, an anti-C5 antibody binds to an epitope within a fragment of the beta chain (SEQ ID NO: 1) of C5 which comprises at least one amino acid selected from the group consisting of Thr47, Glu48, Ala49, Phe50, Asp51, Ala52, Thr53, Lys57, His70, Val71, His72, Ser74, Glu76, Val107, Ser108, Lys109, and His110. In certain embodiments, an anti-C5 antibody binds to an epitope within a fragment of the beta chain (SEQ ID NO: 1) of C5 which comprises at least one amino acid selected from the group consisting of Glu48, Asp51, His70, His72, Lys109, and His110. In certain embodiments, binding of an anti-C5 antibody to a C5 mutant is reduced compared to its binding to wild type C5, wherein the C5 mutant has at least one amino acid substitution at a position selected from the group consisting of Glu48, Asp51, His72, and Lys109. In another embodiment, pH-dependent binding (described below) of an anti-C5 antibody to a C5 mutant is reduced compared to its pH-dependent binding to wild type C5, wherein the C5 mutant has at least one amino acid substitution at a position selected from the group consisting of His70, His72, and His110. In a further embodiment, an amino acid at a position selected from Glu48, Asp51, and Lys109 is substituted with alanine, and an amino acid at a position selected from His70, His72, and His110 is substituted with tyrosine in the C5 mutant.

In another aspect, the anti-C5 antibodies for the present invention may exhibit pH-dependent binding characteristics. As used herein, the expression "pH-dependent binding" means that the antibody exhibits "reduced binding to C5 at acidic pH as compared to its binding at neutral pH" (for purposes of the present disclosure, both expressions may be used interchangeably). For example, antibodies "with pH-dependent binding characteristics" include antibodies that bind to C5 with higher affinity at neutral pH than at acidic pH. In certain embodiments, the antibodies bind to C5 with at least 2, 3, 5, 10, 15, 20, 25, 30, 35, 40, 45, 50, 55, 60, 65, 70, 75, 80, 85, 90, 95, 100, 200, 400, 1000, 10000, or more times higher affinity at neutral pH than at acidic pH. In some embodiments, the antibodies bind to C5 with higher affinity at pH7.4 than at pH5.8. In further embodiments, the antibodies bind to C5 with at least 2, 3, 5, 10, 15, 20, 25, 30, 35, 40, 45, 50, 55, 60, 65, 70, 75, 80, 85, 90, 95, 100, 200, 400, 1000, 10000, or more times higher affinity at pH7.4 than at pH5.8.

The "affinity" of an antibody for C5, for purposes of the present disclosure, is expressed in terms of the KD of the antibody. The KD of an antibody refers to the equilibrium dissociation constant of an antibody-antigen interaction. The greater the KD value is for an antibody binding to its antigen, the weaker its binding affinity is for that particular antigen. Accordingly, as used herein, the expression "higher affinity at neutral pH than at acidic pH" (or the equivalent expression "pH-dependent binding") means that the KD for the antibody binding to C5 at acidic pH is greater than the KD for the antibody binding to C5 at neutral pH. For example, in the context of the present invention, an antibody is considered to bind to C5 with a higher affinity at neutral pH than at acidic pH if the KD of the antibody binding to C5 at acidic pH is at least 2 times greater than the KD of the antibody binding to C5 at neutral pH. Thus, the anti-C5 antibody for the present invention includes antibodies that bind to C5 at acidic pH with a KD that is at least 2, 3, 5, 10, 15, 20, 25, 30, 35, 40, 45, 50, 55, 60, 65, 70, 75, 80, 85, 90, 95, 100, 200, 400, 1000, 10000, or more times greater than the KD of the antibody binding to C5 at neutral pH. In another embodiment, the KD value of the antibody at neutral pH can be 10⁻⁷ M, 10⁻⁸ M, 10⁻⁹ M, 10⁻¹⁰ M, 10⁻¹¹ M, 10⁻¹² M, or less. In another embodiment, the KD value of the antibody at acidic pH can be 10⁻⁹ M, 10⁻⁸ M, 10⁻⁷ M, 10⁻⁶ M, or greater.

In further embodiments an antibody is considered to bind to C5 with a higher affinity at neutral pH than at acidic pH if the KD of the antibody binding to C5 at pH5.8 is at least 2 times greater than the KD of the antibody binding to C5 at pH7.4. In some embodiments the antibodies bind to C5 at pH5.8 with a KD that is at least 3, 5, 10, 15, 20, 25, 30, 35, 40, 45, 50, 55, 60, 65, 70, 75, 80, 85, 90, 95, 100, 200, 400, 1000, 10000, or more times greater than the KD of the antibody binding to C5 at pH7.4. In another embodiment, the KD value of the antibody at pH7.4 can be 10⁻⁷ M, 10⁻⁸ M, 10⁻⁹ M, 10⁻¹⁰ M, 10⁻¹¹ M, 10⁻¹² M, or less. In another embodiment, the KD value of the antibody at pH5.8 can be 10⁻⁹ M, 10⁻⁸ M, 10⁻⁷ M, 10⁻⁶ M, or greater.

The binding properties of an antibody for a particular antigen may also be expressed in terms of the kd of the antibody. The kd of an antibody refers to the dissociation rate constant of the antibody with respect to a particular antigen and is expressed in terms of reciprocal seconds (i.e., sec⁻¹). An increase in kd value signifies weaker binding of an antibody to its antigen. The present invention therefore includes antibodies that bind to C5 with a higher kd value at acidic pH than at neutral pH. The antibodies for the present invention includes antibodies that bind to C5 at acidic pH with a kd that is at least 2, 3, 5, 10, 15, 20, 25, 30, 35, 40, 45, 50, 55, 60, 65, 70, 75, 80, 85, 90, 95, 100, 200, 400, 1000, 10000, or more times greater than the kd of the antibody binding to C5 at neutral pH. In another embodiment, the kd value of the antibody at neutral pH can be 10⁻² 1/s, 10⁻³ 1/s, 10⁻⁴ 1/s, 10⁻⁵ 1/s, 10⁻⁶ 1/s, or less. In another embodiment, the kd value of the antibody at acidic pH can be 10⁻³ 1/s, 10⁻² 1/s, 10⁻¹ 1/s, or greater. The antibodies for the present invention also include antibodies that bind to C5 with a higher kd value at pH5.8 than at pH7.4. The antibodies for the present invention include antibodies that bind to C5 at pH5.8 with a kd that is at least 3, 5, 10, 15, 20, 25, 30, 35, 40, 45, 50, 55, 60, 65, 70, 75, 80, 85, 90, 95, 100, 200, 400, 1000, 10000, or more times greater than the kd of the antibody binding to C5 at pH7.4. In another embodiment, the kd value of the antibody at pH7.4 can be 10⁻² 1/s, 10⁻³ 1/s, 10⁻⁴ 1/s, 10⁻⁵ 1/s, 10⁻⁶ 1/s, or less. In another embodiment, the kd value of the antibody at pH5.8 can be 10⁻³ 1/s, 10⁻² 1/s, 10⁻¹ 1/s, or greater.

In certain instances, a "reduced binding to C5 at acidic pH as compared to its binding at neutral pH" is expressed in terms of the ratio of the KD value of the antibody binding to C5 at acidic pH to the KD value of the antibody binding to C5 at neutral pH (or vice versa). For example, an antibody may be regarded as exhibiting "reduced binding to C5 at acidic pH as compared to its binding at neutral pH", for purposes of the present invention, if the antibody exhibits an acidic/neutral KD ratio of 2 or greater. In certain exemplary embodiments, the pH5.8/pH7.4 KD ratio for an antibody is 2 or greater. In certain exemplary embodiments, the acidic/neutral KD ratio for an antibody can be 2, 3, 5, 10, 15, 20, 25, 30, 35, 40, 45, 50, 55, 60, 65, 70, 75, 80, 85, 90, 95, 100, 200, 400, 1000, 10000, or greater. In another embodiment, the KD value of the antibody at neutral pH can be 10⁻⁷ M, 10⁻⁸ M, 10⁻⁹ M, 10⁻¹⁰ M, 10⁻¹¹ M, 10⁻¹² M, or less. In another embodiment, the KD value of the antibody at acidic pH can be 10⁻⁹ M, 10⁻⁸ M, 10⁻⁷ M, 10⁻⁶ M, or greater. In further instances an antibody may be regarded as exhibiting "reduced binding to C5 at acidic pH as compared to its binding at neutral pH", for purposes of the present invention, if the antibody exhibits a pH5.8/pH7.4 KD ratio of 2 or greater. In certain exemplary embodiments, the pH5.8/pH7.4 KD ratio for an antibody can be 3, 5, 10, 15, 20, 25, 30, 35, 40, 45, 50, 55, 60, 65, 70, 75, 80, 85, 90, 95, 100, 200, 400, 1000, 10000, or greater. In another embodiment, the KD value of the antibody at pH7.4 can be 10⁻⁷ M, 10⁻⁸ M, 10⁻⁹ M, 10⁻¹⁰ M, 10⁻¹¹ M, 10⁻¹² M, or less. In another embodiment, the KD value of the antibody at pH5.8 can be 10⁻⁹ M, 10⁻⁸ M, 10⁻⁷ M, 10⁻⁶ M, or greater.

In certain instances, a "reduced binding to C5 at acidic pH as compared to its binding at neutral pH" is expressed in terms of the ratio of the kd value of the antibody binding to C5 at acidic pH to the kd value of the antibody binding to C5 at neutral pH (or vice versa). For example, an antibody may be regarded as exhibiting "reduced binding to C5 at acidic pH as compared to its binding at neutral pH", for purposes of the present invention, if the antibody exhibits an acidic/neutral kd ratio of 2 or greater. In certain exemplary embodiments, the pH5.8/pH7.4 kd ratio for an antibody is 2 or greater. In certain exemplary embodiments, the acidic/neutral kd ratio for an antibody can be 2, 3, 5, 10, 15, 20, 25, 30, 35, 40, 45, 50, 55, 60, 65, 70, 75, 80, 85, 90, 95, 100, 200, 400, 1000, 10000, or greater. In further exemplary embodiments, the pH 5.8/pH 7.4 kd ratio for an antibody can be 2, 3, 5, 10, 15, 20, 25, 30, 35, 40, 45, 50, 55, 60, 65, 70, 75, 80, 85, 90, 95, 100, 200, 400, 1000, 10000, or greater. In another embodiment, the kd value of the antibody at neutral pH can be 10⁻² 1/s, 10⁻³ 1/s, 10⁻⁴ 1/s, 10⁻⁵ 1/s, 10⁻⁶ 1/s, or less. In a further embodiment, the kd value of the antibody at pH 7.4 can be 10⁻² 1/s, 10 ⁻³ 1/s, 10⁻⁴ 1/s, 10⁻⁵ 1/s, 10⁻⁶ 1/s, or less. In another embodiment, the kd value of the antibody at acidic pH can be 10⁻³ 1/s, 10⁻² 1/s, 10⁻⁹ 1/s, or greater. In a further embodiment, the kd value of the antibody at pH5.8 can be 10⁻³ 1/s, 10⁻² 1/s, 10⁻¹ 1/s, or greater.

As used herein, the expression "acidic pH" means a pH of 4.0 to 6.5. The expression "acidic pH" includes pH values of any one of 4.0, 4.1, 4.2, 4.3, 4.4, 4.5, 4.6, 4.7, 4.8, 4.9, 5.0, 5.1, 5.2, 5.3, 5.4, 5.5, 5.6, 5.7, 5.8, 5.9, 6.0, 6.1, 6.2, 6.3, 6.4, and 6.5. In particular aspects, the "acidic pH" is 5.8.

As used herein, the expression "neutral pH" means a pH of 6.7 to about 10.0. The expression "neutral pH" includes pH values of any one of 6.7, 6.8, 6.9, 7.0, 7.1, 7.2, 7.3, 7.4, 7.5, 7.6, 7.7, 7.8, 7.9, 8.0, 8.1, 8.2, 8.3, 8.4, 8.5, 8.6, 8.7, 8.8, 8.9, 9.0, 9.1, 9.2, 9.3, 9.4, 9.5, 9.6, 9.7, 9.8, 9.9, and 10.0. In particular aspects, the "neutral pH" is 7.4.

KD values, and kd values, as expressed herein, may be determined using a surface plasmon resonance-based biosensor to characterize antibody-antigen interactions. KD values and kd values can be determined at 25 degrees C or 37 degrees C.

In another aspect, an anti-C5 antibody comprises a heavy chain variable domain (VH) sequence having at least 90%, 91%, 92%, 93%, 94%, 95%, 96%, 97%, 98%, 99%, or 100% sequence identity to the amino acid sequence of SEQ ID NO: 2. In certain embodiments, the VH sequence is the amino acid sequence of SEQ ID NO: 2. In certain embodiments, a VH sequence having at least 90%, 91%, 92%, 93%, 94%, 95%, 96%, 97%, 98%, or 99% identity contains substitutions (e.g., conservative substitutions), insertions, or deletions relative to the reference sequence, but an anti-C5 antibody comprising that sequence retains the ability to bind to C5. In certain embodiments, a total of 1 to 10 amino acids have been substituted, inserted and/or deleted in SEQ ID NO: 2. In certain embodiments, substitutions, insertions, or deletions occur in regions outside the HVRs (i.e., in the FRs). Optionally, the anti-C5 antibody comprises the VH sequence in SEQ ID NO: 2, including post-translational modifications of that sequence. In a particular embodiment, the VH comprises one, two or three HVRs selected from: (a) a HVR-H1 comprising the amino acid sequence of SEQ ID NO: 3, (b) a HVR-H2 comprising the amino acid sequence of SEQ ID NO: 4, and (c) a HVR-H3 comprising the amino acid sequence of SEQ ID NO: 5.

As used herein, the term "HVR" stands for a "hypervariable region" and the term "FR" stands for a "framework".

"Framework" or "FR" refers to variable domain residues other than hypervariable region (HVR) residues. The FR of a variable domain generally consists of four FR domains: FR1, FR2, FR3, and FR4. Accordingly, the HVR and FR sequences generally appear in the following sequence in VH (or VL): FR1-H1(L1)-FR2-H2(L2)-FR3-H3(L3)-FR4.

The term "hypervariable region" or "HVR" as used herein refers to each of the regions of an antibody variable domain which are hypervariable in sequence ("complementarity determining regions" or "CDRs") and/or form structurally defined loops ("hypervariable loops") and/or contain the antigen-contacting residues ("antigen contacts"). Generally, antibodies comprise six HVRs: three in the VH (H1, H2, H3), and three in the VL (L1, L2, L3). Exemplary HVRs herein include: (a) hypervariable loops occurring at amino acid residues 26-32 (L1), 50-52 (L2), 91-96 (L3), 26-32 (H1), 53-55 (H2), and 96-101 (H3) (Chothia, J. Mol. Biol. 196:901-917 (1987));(b) CDRs occurring at amino acid residues 24-34 (L1), 50-56 (L2), 89-97 (L3), 31-35b (H1), 50-65 (H2), and 95-102 (H3) (Kabat et al., Sequences of Proteins of Immunological Interest, 5th Ed. Public Health Service, NIH, Bethesda, MD (1991));(c) antigen contacts occurring at amino acid residues 27c-36 (L1), 46-55 (L2), 89-96 (L3), 30-35b (H1), 47-58 (H2), and 93-101 (H3) (MacCallum et al. J. Mol. Biol. 262:732-745 (1996)); and(d) combinations of (a), (b), and/or (c), including HVR amino acid residues 46-56 (L2), 47-56 (L2), 48-56 (L2), 49-56 (L2), 26-35 (H1), 26-35b (H1), 49-65 (H2), 93-102 (H3), and 94-102 (H3). The HVR residues and other residues in the variable domain (e.g., FR residues) are numbered herein according to Kabat et al., supra.

In another aspect, an anti-C5 antibody comprises a light chain variable domain (VL) having at least 90%, 91%, 92%, 93%, 94%, 95%, 96%, 97%, 98%, 99%, or 100% sequence identity to the amino acid sequence of SEQ ID NO: 6. In certain embodiments, the VL sequence is the amino acid sequence of SEQ ID NO: 6. In certain embodiments, a VL sequence having at least 90%, 91%, 92%, 93%, 94%, 95%, 96%, 97%, 98%, or 99% identity contains substitutions (e.g., conservative substitutions), insertions, or deletions relative to the reference sequence, but an anti-C5 antibody comprising that sequence retains the ability to bind to C5. In certain embodiments, a total of 1 to 10 amino acids have been substituted, inserted and/or deleted in SEQ ID NO: 6. In certain embodiments, the substitutions, insertions, or deletions occur in regions outside the HVRs (i.e., in the FRs). Optionally, the anti-C5 antibody comprises the VL sequence in SEQ ID NO: 6, including post-translational modifications of that sequence. In a particular embodiment, the VL comprises one, two or three HVRs selected from (a) a HVR-L1 comprising the amino acid sequence of SEQ ID NO: 7; (b) a HVR-L2 comprising the amino acid sequence of SEQ ID NO: 8; and (c) a HVR-L3 comprising the amino acid sequence of SEQ ID NO: 9.

In another embodiment, the antibody is a full length antibody, e.g., an intact IgG1, IgG2, IgG3 or IgG4 antibody, or an antibody engineered to have regions from two or more IgGs selected from IgG1, IgG2, IgG3, and IgG4 within the region(s) homologous among IgG subclasses by recombination. The antibody may comprise any suitable Fc region comprising a human Fc region sequence (e.g., a human IgG1, IgG2, IgG3 or IgG4 Fc region).

### Fc region variants

In certain embodiments, an anti-C5 antibody for the present invention comprises an Fc region variant in which one or more amino acid modifications have been introduced into a native sequence Fc region of an antibody. The Fc region variant may comprise a human Fc region sequence (e.g., a human IgG1, IgG2, IgG3 or IgG4 Fc region) comprising an amino acid modification (e.g., a substitution) at one or more amino acid positions.

In certain embodiments, the invention contemplates an antibody variant that possesses some but not all effector functions, which make it a desirable candidate for applications in which the half-life of the antibody in vivo is important yet certain effector functions (such as complement and ADCC) are unnecessary or deleterious. In vitro and/or in vivo cytotoxicity assays can be conducted to confirm the reduction/ depletion of CDC and/or ADCC activities. For example, Fc receptor (FcR) binding assays can be conducted to ensure that the antibody lacks Fc gamma R binding (hence likely lacking ADCC activity), but retains FcRn binding ability. The primary cells for mediating ADCC, NK cells, express Fc gamma RIII only, whereas monocytes express Fc gamma RI, Fc gamma RII and Fc gamma RIII. FcR expression on hematopoietic cells is summarized in Table 3 on page 464 of Ravetch and Kinet, Annu. Rev. Immunol. 9:457-492 (1991). Non-limiting examples of in vitro assays to assess ADCC activity of a molecule of interest is described in US Patent No. 5,500,362 (see, e.g., Hellstrom et al., Proc. Nat'l Acad. Sci. USA 83:7059-7063 (1986)) and Hellstrom et al., Proc. Nat'l Acad. Sci. USA 82:1499-1502 (1985); US Pat. No. 5,821,337 (see Bruggemann et al., J. Exp. Med. 166:1351-1361 (1987)). Alternatively, non-radioactive assays methods may be employed (see, for example, ACTI^{™} non-radioactive cytotoxicity assay for flow cytometry (CellTechnology, Inc. Mountain View, CA); and CytoTox 96 (registered trademark) non-radioactive cytotoxicity assay (Promega, Madison, WI)). Useful effector cells for such assays include peripheral blood mononuclear cells (PBMC) and Natural Killer (NK) cells. Alternatively, or additionally, ADCC activity of the molecule of interest may be assessed in vivo, e.g., in an animal model such as that disclosed in Clynes et al., Proc. Nat'l Acad. Sci. USA 95:652-656 (1998). C1q binding assays may also be carried out to confirm that the antibody is unable to bind C1q and hence lacks CDC activity. See, e.g., C1q and C3c binding ELISA in WO 2006/029879 and WO 2005/100402. To assess complement activation, a CDC assay may be performed (see, for example, Gazzano-Santoro et al., J. Immunol. Methods 202:163 (1996); Cragg et al., Blood 101:1045-1052 (2003); and Cragg et al., Blood 103:2738-2743 (2004)). FcRn binding and in vivo clearance/ half-life determinations can also be performed using methods known in the art (see, e.g., Petkova et al., Int'l. Immunol. 18(12):1759-1769 (2006)).

Antibodies with reduced effector function include those with substitution of one or more of Fc region residues 238, 265, 269, 270, 297, 327 and 329 (US Patent No. 6,737,056). Such Fc mutants include Fc mutants with substitutions at two or more of amino acid positions 265, 269, 270, 297 and 327, including the so-called "DANA" Fc mutant with substitution of residues 265 and 297 to alanine (US Patent No. 7,332,581).

Certain antibody variants with improved or diminished binding to FcRs are described. (See, e.g., US Patent No. 6,737,056; WO 2004/056312, and Shields et al., J. Biol. Chem. 9(2):6591-6604 (2001).)

In certain embodiments, an antibody variant comprises an Fc region with one or more amino acid substitutions which improve ADCC, e.g., substitutions at positions 298, 333, and/or 334 of the Fc region (EU numbering of residues).

In some embodiments, alterations are made in the Fc region that result in altered (i.e., either improved or diminished) C1q binding and/or Complement Dependent Cytotoxicity (CDC), e.g., as described in US Patent No. 6,194,551, WO 1999/51642, and Idusogie et al., J. Immunol. 164:4178-4184 (2000).

Antibodies with increased half-lives and improved binding to the neonatal Fc receptor (FcRn), which is responsible for the transfer of maternal IgGs to the fetus (Guyer et al., J. Immunol. 117:587 (1976) and Kim et al., J. Immunol. 24:249 (1994)), are described in US2005/0014934 (Hinton et al.). Those antibodies comprise an Fc region with one or more substitutions therein which improve binding of the Fc region to FcRn. Such Fc variants include those with substitutions at one or more of Fc region residues: 238, 256, 265, 272, 286, 303, 305, 307, 311, 312, 317, 340, 356, 360, 362, 376, 378, 380, 382, 413, 424 or 434, e.g., substitution of Fc region residue 434 (US Patent No. 7,371,826).

See also Duncan, Nature 322:738-40 (1988); US Patent No. 5,648,260; US Patent No. 5,624,821; and WO 1994/29351 concerning other examples of Fc region variants.

In certain embodiments, an anti-C5 antibody for the present invention comprises a VH as in any of the embodiments provided above and a heavy chain constant region comprising the amino acid sequence of any one of SEQ ID NOs: 10, 11, 12, 13, 14, and 15. In certain embodiments, an anti-C5 antibody comprises a VL as in any of the embodiments provided above and a light chain constant region comprising the amino acid sequence of any one of SEQ ID NOs: 16, 17, and 18.

In certain embodiments, an anti-C5-antibody for the present invention is any one of the antibodies described in WO2016/098356, WO2017/123636, and WO2017/132259.

### Subcutaneous administration

The composition for use of the present invention is administered subcutaneously in two phases. In both phases, there is an interval between every two subcutaneous administrations. Each phase comprises at least one interval. The last interval of the first phase is between the last subcutaneous administration of the first phase and the first subcutaneous administration of the second phase.

In certain embodiments, the at least one interval in the first phase is 1 day to 2 months. An adequate interval in the first phase can be determined within a suitable range according to conditions of a subject or a patient. Specific administration interval between subcutaneous injections is 3 days, 4 days, 5 days, 6 days, 7 days, 8 days, 9 days, 10 days, 11 days, 12 days, 13 days, 14 days, 15 days, 16 days, 17 days, 18 days, 19 days, 20 days, 21 days, 22 days, 23days, 24 days, 25 days, 26 days, 27 days, 28 days, 29 days, 30 days, 31 days, 32 days, 33 days, 34 days, 35 days, 36 days, 37 days, 38 days, 39 days, 40 days, 41 days, or 42 days. In preferred embodiments, the interval in the first phase is 4 days to 35 days. In further preferred embodiments, the interval in the first phase is 5 days to 14 days. In the most preferred embodiments, the interval in the first phase is 7 or 14 days. The pharmaceutical composition may be administered once every week (weekly), or once every two weeks (two-weekly or biweekly) in the first phase.

In certain embodiments, the at least one interval in the second phase is between 2 days and 6 months. An adequate length of the at least one interval in the second phase can be determined within a suitable range according to conditions of a subject or a patient. An exemplified length of the at least one interval in the second phase is 2 days, 3 days, 4 days, 5 days, 6 days, 7 days, 8 days, 9 days, 10 days, 11 days, 12 days, 13 days, 14 days, 15 days, 16 days, 17 days, 18 days, 19 days, 20 days, 21 days, 22 days, 23days, 24 days, 25 days, 26 days, 27 days, 28 days, 29 days, 30 days, 31 days, 32 days, 33 days, 34 days, 35 days, 36 days, 37 days, 38 days, 39 days, 40 days, 41 days, 42 days, 43 days, 44 days, 45 days, 46 days, 47 days, 48 days, 49 days, 50 days, 51 days, 52 days, 53 days, 54 days, 55 days, 56 days, 57 days, 58 days, 59 days, 60 days, 61 days, 62 days, 63 days, 64 days, 65 days, 66 days, 67 days, 68 days, 69 days, 70 days, 71 days, 72 days, 73 days, 74 days, 75 days, 76 days, 77 days, 78 days, 79 days, 80 days, 81 days, 82 days, 83 days, 84 days, 85 days, 86 days, 87 days, 88 days, 89 days, 90 days, 91 days, 92 days, 93 days, 94 days, 95 days, 96 days, 97 days, 98 days, 99 days, 100 days, 101 days, 102 days, 103 days, 104 days, 105 days, 106 days, 107 days, 108 days, 109 days, 110 days, 111 days, 112 days, 113 days, 114 days, 115 days, 116 days, 117 days, 118 days, 119 days, 120 days, 121 days, 122 days, 123 days, 124 days, 125 days, 126 days, 127 days, 128 days, 129 days, 130 days, 131 days, 132 days, 133 days, 134 days, 135 days, 136 days, 137 days, 138 days, 139 days, 140 days, 141 days, 142 days, 143 days, 144 days, 145 days, 146 days, 147 days, 148 days, 149 days, 150 days, 151 days, 152 days, 153 days, 154 days, 155 days, 156 days, 157 days, 158 days, 159 days, 160 days, 161 days, 162 days, 163 days, 164 days, 165 days, 166 days, 167 days, 168 days, 169 days, 170 days, 171 days, 172 days, 173 days, 174 days, 175 days, 176 days, 177 days, 178 days, 179 days, 180 days, 181 days, 182 days, 183 days, or 184 days. In preferred embodiments, the length of the at least one interval of the second phase is 15 days to 3 months. In the second phase, the pharmaceutical composition may be administered once every month (monthly), once every two months (two-monthly or bimonthly), or once every three months (three-monthly or trimonthly). The longer term can reduce patients' burden or suffering.

In certain embodiments, the pharmaceutical composition is administered subcutaneously at a dose of 17 to 6,000 mg of the antibody in the first and the second phases. An adequate dose can be determined within a suitable range according to conditions of a subject or a patient. In case that the pharmaceutical composition is administered subcutaneously to a subject repeatedly, the doses do not have to be the same all the time and may be determined within the suitable range. For example, the dose may be decreased gradually.

In certain embodiments, the dose in the subcutaneous administration of the first phase is 50 to 350 mg of the antibody. When the antibody dose per subcutaneous administration in the first phase is lower than the antibody dose per subcutaneous administration in the second phase, the dose of the antibody per subcutaneous administration in the first phase is preferably 150 to 200 mg. A specific preferred dose include 170 mg in this case. When the antibody dose per subcutaneous administration in the first phase is the same as the antibody dose per subcutaneous administration in the second phase, the dose per subcutaneous administration in the first phase is preferably 300 to 350 mg of the antibody. A specific preferred dose include 340 mg in this case. When the pharmaceutical composition is administered weekly (with about 7 days of interval) in the first phase, a preferred dose is 170 mg or 340 mg. When the pharmaceutical composition is administered two-weekly (with about 14 days of interval) in the first phase, a preferred dose is 340 mg.

In certain embodiments, the dose of the antibody per administration in the second phase is 350 mg to 1,000 mg. In preferred embodiments, the dose is 650 to 700 mg. When the pharmaceutical composition is administered four-weekly (with about 28 days of interval) or monthly, a preferred dose is 680 mg. The dosing regimen of a subcutaneous administration of 680 mg antibody four-weekly or monthly would be particularly preferred, as it can reduce patients' burden or suffering.

In certain embodiments, the anti-C5 antibody dose per administration in the first phase is three to five folds lower than the anti-C5 antibody dose per administration in the second phase. In preferred embodiments, the dose of the antibody per subcutaneous administration in the first phase is four folds lower than the antibody dose per subcutaneous administration in the second phase.

In certain embodiments, the number of subcutaneous administrations in the first phase is 1 to 12. In preferred embodiments, the number is 5 times to 10 times. In further preferred embodiments, there are 8 subcutaneous administrations in the first phase.

In certain embodiment, any pharmaceutically acceptable carrier available for an ordinary composition for subcutaneous injection can be included in the pharmaceutical composition. A kind of carrier used for subcutaneous injection can be suitably selected from carriers generally known in the art.

In certain embodiment, a formulation of the pharmaceutical composition can be any one selected from the group consisting of liquid, semisolid, and solid. The solid composition is usually made by lyophilization from a liquid formulation. The lyophilized composition is usually reconstituted using water or saline solution just before subcutaneous injection.

When the composition is administered subcutaneously, a concentration of anti-C5 antibody in a liquid formulation of the composition is determined within an ordinary range in the art. In certain embodiment, a volume of the composition for subcutaneous injection is determined within an ordinary range in the art.

In certain embodiments, the C5-related disease is a complement-mediated disease or condition which involves excessive or uncontrolled activation of C5. In certain embodiments, the C5-related disease is at least one selected from a group consisting of rheumatoid arthritis (RA); lupus nephritis; ischemia-reperfusion injury; paroxysmal nocturnal hemoglobinuria (PNH); atypical hemolytic uremic syndrome (aHUS); dense deposit disease (DDD); macular degeneration; hemolysis, elevated liver enzymes, and low platelets (HELLP) syndrome; thrombotic thrombocytopenic purpura (TTP); spontaneous fetal loss; Pauci-immune vasculitis; epidermolysis bullosa; recurrent fetal loss; multiple sclerosis (MS); traumatic brain injury; and injury resulting from myocardial infarction, cardiopulmonary bypass or hemodialysis; refractory generalized myasthenia gravis (gMG); neuromyelitis optica (NMO). In preferred embodiment, the C5-related disease is at least one selected from a group consisting of PNH, aHUS, gMG and NMO. In further preferred embodiment, the C5-related disease is PNH.

In certain aspects, a pharmaceutical composition formulated for intravenous administration and comprising an anti-C5 antibody is administrated before the first subcutaneous administration of the first phase. Preferably, the anti-C5 antibody of the intravenously administered composition is the same as the anti-C5 antibody of the subcutaneously administered composition. The intravenously administered pharmaceutical composition is the same as referred to hereinafter.

In certain embodiments, the composition for subcutaneous injection is subcutaneously administered on the same day as or one or more days after a dose of the composition for intravenous injection is intravenously administered. One or more doses of the composition for intravenous injection may be administered to a subject or a patient before administering a first dose of the composition for subcutaneous injection. In a preferred embodiment, the first dose of the composition for subcutaneous injection is administered after the final dose of the composition for intravenous injection.

In certain embodiments, the first subcutaneous administration of the first phase is administrated 0 days to 1 month after the final administration of the intravenously administrated pharmaceutical composition. Specifically, the period between the first subcutaneous administration and the final intravenous administration is 0 day (i.e., within 24 hours), 1day, 2 days, 3 days, 4 days, 5 days, 6 days, 7 days, 8 days, 9 days, 10 days, 11 days, 12 days, 13 days, 14 days, 15 days, 16 days, 17 days, 18 days, 19 days, 20 days, 21 days, 22 days, 23days, 24 days, 25 days, 26 days, 27 days, 28 days, 29 days, 30 days, or 31 days. In preferred embodiments, the period is 0 day (i.e., within 24 hours) to 14 days. In further preferred embodiments, the period is 0 day (i.e., within 24 hours) to 10 days. In the most preferred embodiments, the period is 0 day (i.e., within 24 hours) to 8 days.

### III. A pharmaceutical composition for intravenous injection

In certain aspects of the invention, the first subcutaneous administration in the first phase of the pharmaceutical composition for use of the present invention is administered after the final administration of a composition for intravenous administration.

Therefore, in one embodiment of the present invention a composition formulated for intravenous administration and comprising an anti-C5 antibody is administered intravenously before the first subcutaneous administration of the first phase. In a preferred embodiment, the anti-C5 antibody of the intravenous composition is the same as the anti-C5 antibody of the subcutaneous composition.

Preferably, there are no intravenous administrations after the first subcutaneous administration. Therefore, in a further embodiment, the last intravenous administration is before the first subcutaneous administration.

The pharmaceutical composition formulated for intravenous injection in the present invention is for use in treatment or prevention of a C5-related disease, comprises an anti-C5 antibody, and is administered intravenously. The intravenous injection can be carried out using ordinary devices and methods to administer a pharmaceutical composition to vein by injection. Specific devices and methods for the intravenous injection also may be selected.

The anti-C5 antibody used for intravenous injection can be any antibody as described above for pharmaceutical compositions for subcutaneous injection. The pharmaceutical composition for intravenous injection and the pharmaceutical composition for subcutaneous injection may contain the same anti-C5 antibody or may contain different anti-C5 antibody. In preferred embodiments, the anti-C5 antibody used for intravenous injection is the same antibody as for subcutaneous injection set forth above.

In certain embodiments, the pharmaceutical composition is administered intravenously at a dose of 50 to 5,000 mg of the antibody. An adequate dose can be determined within a suitable range according to conditions of a subject or a patient. In case that the pharmaceutical composition is administered intravenously to a subject repeatedly, the doses do not have to be the same all the time and may be determined arbitrarily as long as the doses are within the suitable range. For example, the dose may be decreased gradually. In preferred embodiments, the range of the dose is 55 to 4,000 mg of the antibody. In further preferred embodiments, the range of the dose is 60 to 2,500 mg of the antibody. In the most preferred embodiments, the range of the dose is 100 to 2,000 mg of the antibody. Specific preferred doses are 75 mg, 125 mg, 150 mg, 300 mg, 375 mg, 500 mg and 1,000 mg. Further preferred doses are 375 mg, 500 mg and 1,000 mg, and among these, the most preferred is 1,000 mg.

In certain embodiments, the number of times that the pharmaceutical composition is administered by intravenous injection is not particularly limited and may be once or more times. In preferred embodiments, the number of times is once, twice, or three times. In further preferred embodiments, the number of times is once or twice. In the most preferred embodiment, the number of times is once. The lesser number of times can reduce patients' burden or suffering.

In certain embodiments, in case that the pharmaceutical composition is administered intravenously to a subject or a patient repeatedly, the composition is administered once an hour to once every 14 days. An adequate administration interval between intravenous injections can be determined within a suitable range according to conditions of a subject or a patient. Specific administration interval between intravenous injections is one hour, 2 hours, 3 hours, 4 hours, 5 hours, 6 hours, 7 hours, 8 hours, 9 hours, 10 hours, 11 hours, 12 hours, 13 hours, 14 hours, 15 hours, 16 hours, 17 hours, 18 hours, 19 hours, 20 hours, 21 hours, 22 hours, 23hours, 24 hours, 25 hours, 26 hours, 27 hours, 28 hours, 29 hours, 30 hours, 31 hours, 32 hours, 33 hours, 34 hours, 35 hours, 36 hours, 37 hours, 38 hours, 39 hours, 40 hours, 41 hours, 42 hours, 43 hours, 44 hours, 45 hours, 46 hours, 47 hours, 48 hours, 3 days, 4 days, 5 days, 6 days, 7 days, 8 days, 9 days, 10 days, 11 days, 12 days, 13 days, or 14 days. In preferred embodiments, the administration interval is 24 hours to 10 days. In further preferred embodiments, the administration interval is 48 hours to 7 days. In the most preferred embodiments, the administration interval is 3 to 5 days.

In certain embodiment, any pharmaceutically acceptable carrier available for an ordinary composition for intravenous injection can be included in the composition. A kind of carrier used for intravenous injection can be suitably selected from carriers generally known in the art.

In certain embodiments, a formulation of the pharmaceutical composition can be any one selected from the group consisting of liquid, semisolid, and solid. The sold composition is usually made by lyophilization from a liquid formulation. The lyophilized composition is usually reconstituted using water or saline solution just before intravenous injection. The formulation of the pharmaceutical composition for intravenous injection can be the same as or different from the formulation of the pharmaceutical composition for subcutaneous injection. In preferred embodiments, the formulation of the pharmaceutical composition for intravenous injection is the same as the formulation of the pharmaceutical composition for subcutaneous injection to drive down manufacturing costs.

When the composition is administered intravenously, a concentration of anti-C5 antibody in a liquid formulation of the composition is determined within an ordinary range in the art. In certain embodiment, a volume of the composition for intravenous injection is determined within an ordinary range in the art.

In certain aspects, a dose of the composition for intravenous injection is administered before an initial dose of another pharmaceutical composition is administered subcutaneously. The pharmaceutical composition for subcutaneous injection is the same one set forth above.

In certain embodiments, the composition for intravenous injection is intravenously administered on the same day as or one or more days before the first dose of the composition for subcutaneous injection is subcutaneously administered. One or more doses of the composition for intravenous injection may be administered to a subject or a patient before administering a first dose of the composition for subcutaneous injection. In a preferred embodiment, the final dose of the composition for intravenous injection is administered before the first dose of the composition for subcutaneous injection.

In certain embodiments, the dose of the composition for intravenous injection is administered on the same day as or 1 day to 1 month before the first dose of the composition for subcutaneous injection. Specific interval between the dose of the composition for intravenous injection and the first dose of the composition for subcutaneous injection is 0 day (i.e., within 24 hours), 1day, 2 days, 3 days, 4 days, 5 days, 6 days, 7 days, 8 days, 9 days, 10 days, 11 days, 12 days, 13 days, 14 days, 15 days, 16 days, 17 days, 18 days, 19 days, 20 days, 21 days, 22 days, 23days, 24 days, 25 days, 26 days, 27 days, 28 days, 29 days, 30 days, or 31 days. In preferred embodiments, the interval is 0 day (i.e., within 24 hours) to 14 days. In further preferred embodiments, the interval is 0 day (i.e., within 24 hours) to 10 days. In the most preferred embodiments, the interval is 0 day (i.e., within 24 hours) to 8 days.

### IV. Switching from other pharmacological product

In another aspect, the above pharmaceutical compositions for subcutaneous or intravenous injection can be useful for treatment or prevention of a C5-related disease in a subject who has been treated with at least one pharmacological product for use in treatment or prevention of the disease once or more times. For example, the pharmaceutical compositions of the present invention may be useful for treating a patient having a C5-related disease who has received prior treatment with at least one pharmacological product for treating or preventing the disease but is expected to better respond to the treatment by the pharmaceutical compositions of the present invention. In such cases, the medication can be switched from the pharmacological product to the pharmaceutical composition of the present invention. In preferred embodiments, an initial dose of the composition for intravenous injection in the present invention is administered after the final dose of the pharmacological product that has been used in the prior treatment.

In certain embodiments, the pharmacological product comprises an active substance which is different from the anti-C5 antibody in the above composition for subcutaneous and intravenous injection. In some embodiments, the active substance of pharmacological product is an siRNA targeting C5 mRNA, or an anti-C5 antibody which is different from the anti-C5 antibody comprised in the above composition for subcutaneous and intravenous injection. In preferred embodiments, the pharmacological product comprises the antibody which is different antibody from ones in the above composition for the subcutaneous and intravenous injection. In the most preferred embodiment, the antibody comprised in the pharmacological product that has been used in the prior treatment is eculizumab or its derivative.

In certain embodiments, an initial dose of the composition for intravenous injection in the present invention is administered on the same day as or one or more days after the final dose of the pharmacological product is administered. Specific interval between the final dose of the pharmacological product and the initial dose of the composition for intravenous injection of the present invention is 0 day, which means that the initial dose of the composition for intravenous injection is administered on the same day as the final dose of the pharmacological product, 1 day, 2 days, 3 days, 4 days, 5 days, 6 days, 7 days, 8 days, 9 days, 10 days, 11 days, 12 days, 13 days, 14 days, 15 days, 16 days, 17 days, 18 days, 19 days, 20 days, or 21 days. In preferred embodiments, the composition for intravenous injection of the present invention is administered 3 or more days after the final dose of the pharmacological product. In more preferred embodiments, the composition for intravenous injection of the present invention is administered 7 or more days after the final dose of the pharmacological product. In further preferred embodiments, the composition for intravenous injection of the present invention is administered 14 or more days after the final dose of the pharmacological product. In the most preferred embodiments, the composition for intravenous injection of the present invention is administered 21 or more days after the final dose of the pharmacological product.

### V. A method for treating or preventing

In other aspects, the invention encompasses methods for treating or preventing a C5-related disease.

In one embodiment, the method comprises subcutaneously administering to the subject a pharmaceutical composition, which is formulated for subcutaneous injection and comprises an anti-C5 antibody, wherein the composition is subcutaneously administered in two phases, wherein in both phases there is an interval between every two subcutaneous administrations, wherein each phase comprises at least one interval, and wherein in the first phase
i) the at least one interval is shorter than the at least one interval in the second phase, and
ii) the dose of the antibody per administration is lower than or the same as the antibody dose per administration in the second phase.
The subcutaneously administered pharmaceutical composition is the same as referred to hereinbefore.

In certain embodiments, a pharmaceutical composition formulated for intravenous administration and comprising an anti-C5 antibody is administrated before the first subcutaneous administration of the first phase. The intravenously administered pharmaceutical composition is the same as referred to hereinbefore.

Conditions and target diseases of a pharmaceutical composition for subcutaneous or intravenous injection used in the method are the same as those mentioned in the above sections "II. A pharmaceutical composition for subcutaneous injection" and "III. A pharmaceutical composition for intravenous injection".

### VI. Vaccination

Infectious disease, e.g. meningococcal infections, can occur in a subject to whom anti-C5 antibody was administered. To prevent such infectious diseases, the subject may be immunized by a vaccine known for treating or preventing the diseases before, after or when the above composition for subcutaneous and intravenous injection is administered.

### VII. Article of manufacture

In another aspect of the invention, an article of manufacture or product containing materials useful for the treatment or prevention of the C5-related diseases described above is provided. The article of manufacture or product comprises one or more containers and a label or package insert on or associated with the containers. Suitable containers include, for example, bottles, vials, syringes, SC solution syringes, etc. The containers may be formed from a variety of materials such as glass or plastic. The container holds a composition which is by itself or combined with another composition effective for treating or preventing the condition and may have a sterile access port (for example the container may be a vial having a stopper pierceable by a hypodermic injection needle). At least one active agent in the composition is an anti-C5 antibody described above. The label, package insert, or such document indicates that the composition is used for treating the condition of choice, for example, any of C5-related diseases as described above.

In certain embodiments, the composition contained in the container of the article of manufacture or product is formulated for subcutaneous injection. The article of manufacture or product in this embodiment may further comprise a package insert indicating the following administration method. The pharmaceutical composition is subcutaneously administered in two phases, wherein in both phases there is an interval between every two subcutaneous administrations. Each phase comprises at least one interval. In the first phase i) the at least one interval is shorter than the at least one interval in the second phase, and ii) the dose of the antibody per administration is lower than the antibody dose per administration in the second phase.

In certain embodiments, the article of manufacture or product may further comprise an additional container with a composition contained therein, wherein the composition comprises a further therapeutic agent. The article of manufacture in this embodiment may further comprise a package insert indicating that the compositions can be used to treat a particular condition. Alternatively, or additionally, the article of manufacture may further comprise another additional container comprising a pharmaceutically-acceptable buffer, such as bacteriostatic water for injection (BWFI), phosphate-buffered saline, Ringer's solution and dextrose solution. It may further include other materials desirable from a commercial and user standpoint, including other buffers, diluents, filters, needles, and syringes.

### Examples

The following are examples of methods and compositions of the invention. It is understood that various other embodiments may be practiced, given the general description provided above.

### Example 1

### Generation of an anti-CS antibody

The anti-C5 antibody 305LO15 in WO2016/098356 was prepared by an ordinary method. Briefly, the genes encoding the heavy chain variable domain (VH) of 305LO15 were combined with the genes encoding a modified human IgG1 heavy chain constant domain (CH) variant SG115 (SEQ ID NO: 13). The genes encoding the light chain variable domain (VL) of 305LO15 were combined with the genes encoding a human light chain constant domain (CL) (SK1, SEQ ID NO: 18). Antibodies were expressed in HEK293 cells co-transfected with the combination of heavy and light chain expression vectors, and were purified by protein A.

### Example 2

Protein pharmaceuticals, e.g. antibody drugs, are typically administered intravenously because proteins are difficult to concentrate. In fact, eculizumab which is the only approved antibody available for treatment at present is administered intravenously. If subcutaneous injection becomes available, it will reduce patient burden, e.g. ambulant treatment and long time spent in hospital for intravenous injection, since self-injection is possible.

To examine the possibility of subcutaneous administration of anti-C5 antibody, 305LO15 disclosed in WO2016/098356 was selected because of its high solubility. It was thought that higher solubility provides the possibility that the protein pharmaceutical can be administered in a smaller volume, allowing subcutaneous injection. Pharmaceutical formulations for intravenous injection and subcutaneous injection were prepared as to be the following general formulation.
170mg/mL 305LO15
30 mM histidine/aspartic acid
100 mM arginine hydrochloride
0.05% Poloxamer 188
pH 5.8

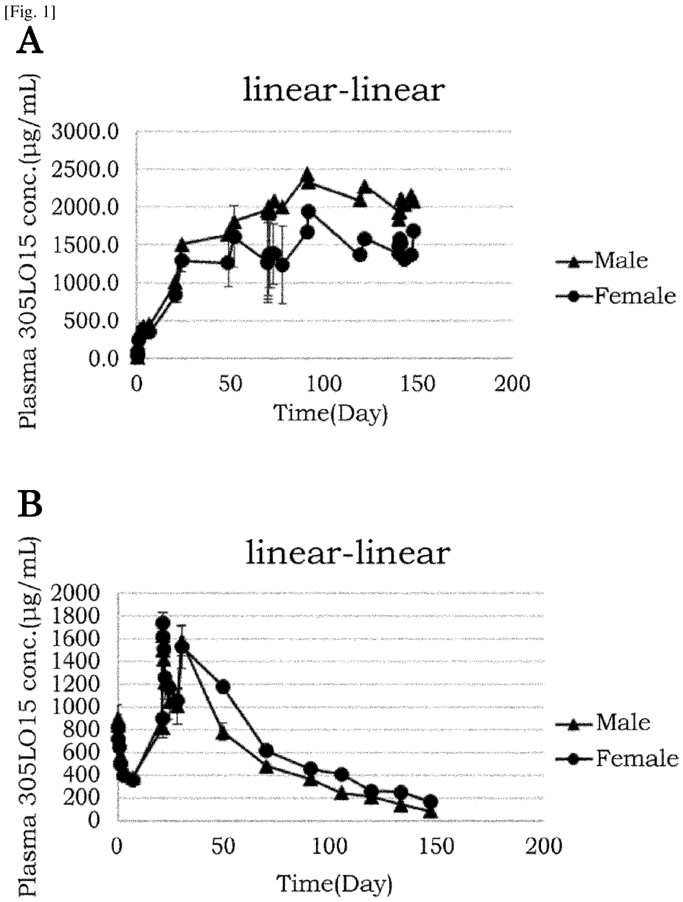

To compare pharmacokinetics of 305LO15 after subcutaneous injection or intravenous injection, cynomolgus monkeys were separated into two groups, a subcutaneous injection group and an intravenous injection group. 305LO15 was administered subcutaneously to the animals of the first group (three males and three females) once every week over twenty-two times in total at 40 mg/kg of the antibody for each dosing. A plasma 305LO15 concentration of those animals was measured using ELISA analysis at the time points shown in Figure 1a. 305LO15 was administered intravenously to the animals of the second group (six males and six females) once every week over five times in total at 40 mg/kg of the antibody for each dosing. A plasma 305LO15 concentration of the animals was measured by ELISA analysis at the time points shown in Figure 1b.

As shown in Figure 1a, the plasma 305LO15 concentration of cynomolgus monkeys increased by subcutaneous injections, verifying that 305LO15 can be administered by subcutaneous injection for therapy. The rate of the initial rise in plasma concentration after the initial dose was lower in the subcutaneous administration (Figure 1a) than in the intravenous injection (Figure 1b).

### Example 3

Next, it was investigated if the relatively slow rate of the rise in plasma concentration after the initial dose of subcutaneous injection can be compensated by an intravenous injection of 305LO15. To five males and five females of cynomolgus monkeys, the initial dose of 305LO15 was administered intravenously once at the dose of 100 mg/kg of the antibody. After a week from the initial intravenous administration, subcutaneous injection of 305LO15 was started. Twenty-six times of the subcutaneous injection were given in total, at the pace of once every week at the dose of 40 mg/kg of the antibody for each dosing.

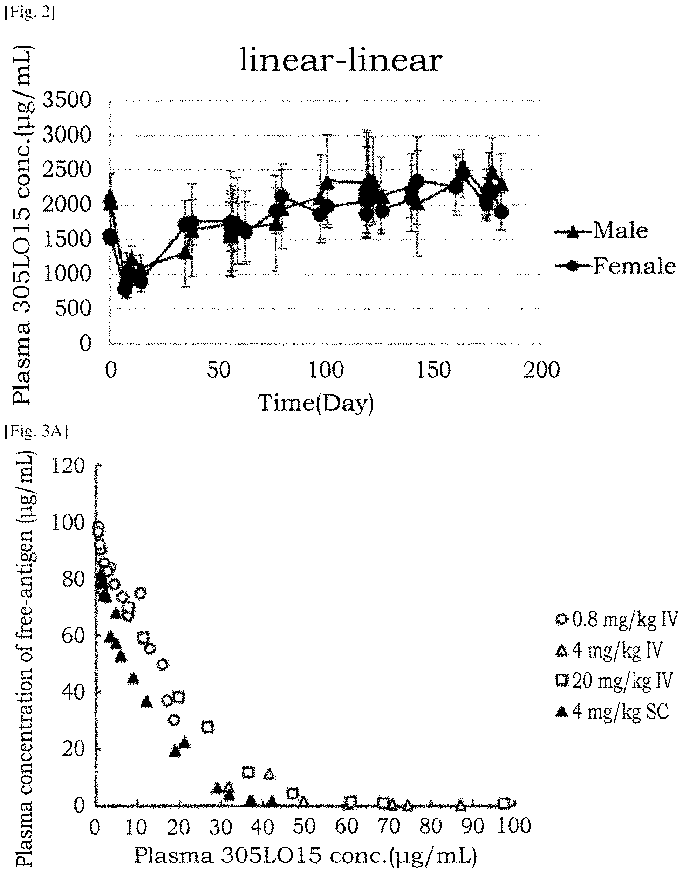

As shown in Figure 2, the rate of the rise in plasma concentration of 305LO15 was faster in the case where the initial dose of the antibody was administered as an intravenous injection before the maintenance doses by subcutaneous injection than in the case of no initial dose of the intravenous injection. The plasma concentration of 305LO15 reached and was maintained at the level comparable to that shown in Figure 1a. It was thought that this faster rise in plasma 305LO15 concentration contributes to faster achievement of therapeutic effect by the drug.

### Example 4

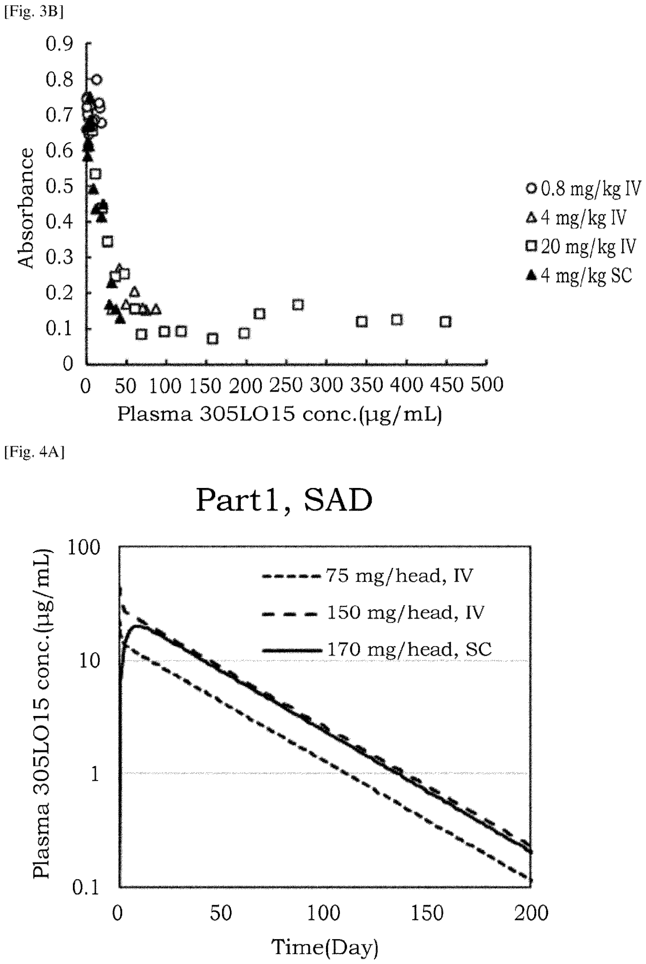

To estimate efficacious plasma concentration of 305LO15, relationship between plasma concentration of the free antigen (C5) or complement activity and plasma concentration of the antibody (305LO15) was determined in cynomolgus monkeys. 305LO15 was administered intravenously (0.8 mg/kg, 4 mg/kg, or 20 mg/kg) or subcutaneously (4 mg/kg). Plasma concentration of the antibody and free C5 was measured by ELISA. Complement activity was measured by RBC hemolytic assay. As shown in Figure 3, free C5 concentration was suppressed to less than sub micro g/mL level when plasma concentration of 305LO15 was maintained at above 40 micro g/mL of 305LO15 (Figure 3a) and 40 micro g/mL or higher plasma concentration of 305LO15 inhibited complement activity (hemolysis) to less than 20% of baseline (i.e. no antibody is applied) (Figure 3b). It was estimated that complement activity is suppressed to less than 20% of baseline (i.e. no antibody is injected) when 40 micro g/ mL or higher concentration of 305LO15 is maintained in plasma.

### Example 5

To determine suitable dosages and administrations for 305LO15 to maintain efficacious plasma 305LO15 concentration for clinical trials, the time course of plasma 305LO15 concentration in humans was predicted by simulation. At first, plasma 305LO15concentrations were measured over time after the antibody was administered to cynomolgus monkeys at 0.8, 4, 20 mg/kg of the antibody for each dosing. Cynomolgus monkey PK parameters were estimated by analyzing the data using a 2-compartment model. Using allometric scaling, human PK parameters were estimated based on the cynomolgus monkey PK parameters. The estimated human PK parameters are shown in Table 1 (abbreviation of parameters in Table 1: BW, body weight; CL, total clearance of drug; F, bioavailability, or a fraction of an administered dose of a drug that becomes systemically available; Ka, absorption rate constant; mAb, monoclonal antibody; PK, pharmacokinetic; Q, intercompartmental clearance; SC, subcutaneous; Vc, volume of distribution for the central compartment; Vp, volume of distribution for the peripheral compartment).

**[Table 1]**

| **Parameter** | **Unit** | **CynomolgusMonkey** | **Human (Allometrically Scaled)** | **Comment** |
|---|---|---|---|---|
| **BW** | kg | 3.5 | 70 | |
| **F** | fraction | 0.91 | 0.75 | Not scaled; typical for a high SC bioavailability of a mAb in human* |
| **Kₐ** | per h | 0.0485 | 0.0125 | Not scaled; typical value for mabs in human* |
| **V_{c}** | mL/kg | 46.6 | 46.6 | Scaled to body weight with an exponent of 1 |
| **Vₚ** | mL/kg | 26.1 | 26.1 | Scaled to body weight with an exponent of 1 |
| **Cl** | mL/h/kg | 0.116 | 0.074 | Scaled to body weight with an exponent of 0.85 |
| **Q** | mL/h/kg | 1.16 | 0.741 | Scaled to body weight with an exponent of 0.85 |

| | | | | |
|---|---|---|---|---|
| * Dirks NL, Meibohm B. Population pharmacokinetics of therapeutic monoclonal antibodies. Clin Pharmacokinet 2010;49:633-59. | | | | |

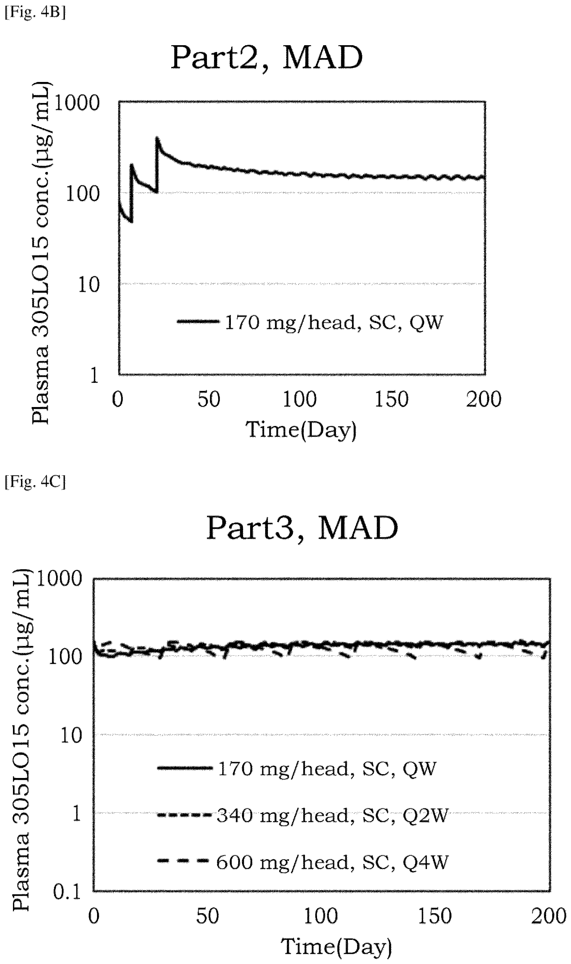

Human PK profile was estimated for each cohort expected for phase 1/2 study (Figure 4). Part 1 of the study was designed to include three groups of subjects. The first group is a group of subjects to whom 305LO15 is administered intravenously once at the dose of 75 mg/body. The second group is a group of subjects to whom 305LO15 is administered intravenously once at the dose of 150 mg/body. The third group is a group of subjects to whom 305LO15 is administered subcutaneously once at the dose of 170 mg/body. It was predicted that plasma 305LO15 concentration in subjects who received any one of the above dose is not maintained above the threshold (40 micro g/ mL) for more than one week (Figure 4a).

Part 2 of the study was designed to include a group of subjects to whom 305LO15 is administered intravenously three times (initially at a dose of 300 mg/body, then at 500 mg/body a week after the initial administration, and finally at 1000 mg/body two weeks after the second administration) and, starting from two weeks after the final intravenous administration, 305LO15 is administered subcutaneously once a week at the dose of 170 mg/body. It was predicted that 305LO15 concentration is maintained higher than the PD threshold (40 micro g/mL) by the dosing regimen for the Part 2 study (Figure 4b).

Part 3 of the study was designed to include three groups of subjects. 305LO15 is initially administered to the subjects of all groups intravenously once at the dose of 500 mg/body. Starting from the day after the initial administration, 305LO15 is administered subcutaneously to subjects of the first group once every week at the dose of 170 mg/body, to subjects of the second group once every two weeks at the dose of 340 mg/body, and to subjects of the third group once every four weeks at the dose of 600 mg/body. It was predicted that 305LO15 concentration is maintained higher than the PD threshold (40 micro g/mL) in all of the three groups in the Part 3 study (Figure 4c).

### Example 6

Eculizumab has been used for treating patients who have PNH or aHUS. In the case that 305LO15 is expected to have a more desirable effect than eculizumab to a PNH or aHUS patient, the drug therapy to the patient may be switched from eculizumab to 305LO15. Meanwhile, there is a possibility that 305LO15 forms immunocomplex with C5 and eculizumab inside the body, because the epitope of 305LO15 is different from that of eculizumab, that is, 305LO15 binds to the beta chain of C5, while eculizumab binds to the alpha chain of C5. Formation of such immunocomplex may cause abnormal activation, and is therefore desirable to be avoided as much as possible.

To investigate if 305LO15 forms immunocomplex with eculizumab (ECZ, heavy chain sequence is shown in SEQ ID NO: 19 and light chain sequence is shown in SEQ ID NO: 20) and human C5 (hC5, SEQ ID NO: 21), in vitro analysis was carried out using size-exclusion chromatography (SEC). hC5 was prepared according to the method disclosed in WO2016/098356. ECZ, hC5, and 305LO15 were dialyzed against DPBS pH7.4 for buffer exchange. Then, ECZ and hC5 were mixed and incubated at 37 degrees C for 3-4 hours. After the incubation, 305LO15 was added to this mixture containing ECZ and hC5. Final concentration of ECZ and hC5 was adjusted to 200 micro g/mL, and 305LO15 concentration was adjusted to 0, 25, 125, 250 or 1250 micro g/mL. After incubating these mixtures at 37 degrees C for 3-4 hours, SEC analysis was carried out at pH7.4 and pH6.0. Specific SEC conditions were as identified below.
HPLC system: Waters Alliance e2695 HPLC system
Column: TSKgel G4000SWXL
Column temp.: 25 degrees C
Eluent: 50 mM Na-PB/300 mM NaCl, pH7.4 or pH6.0
Flow rate: 0.5 mL/min
Detection: 220 nm UV absorption
Injection: 10 micro L

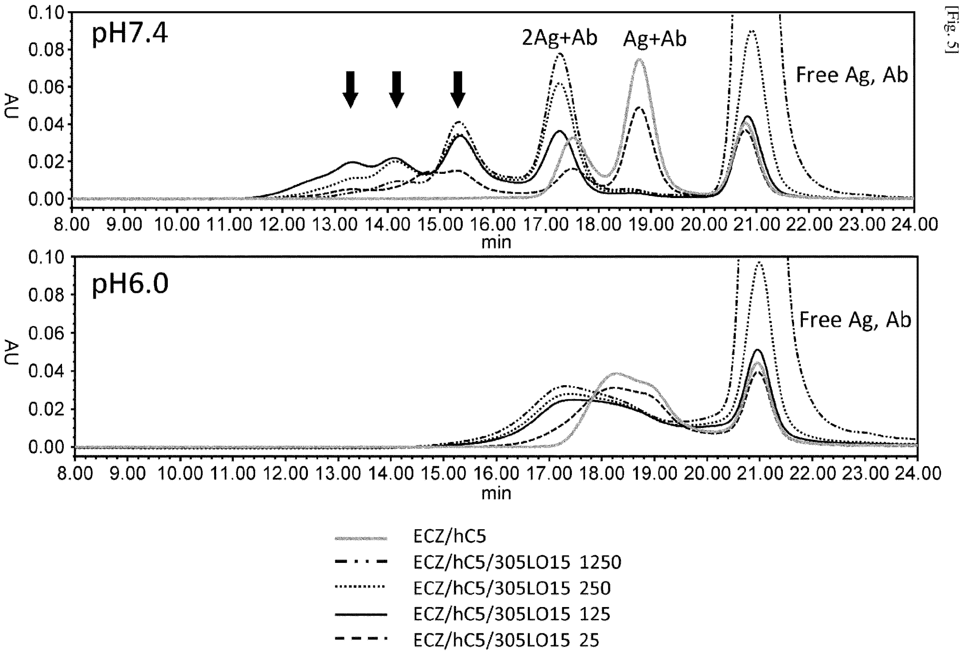

As a result, 305LO15 at the concentrations from 125 to 1250 micro g/mL did not affect the SEC profile at pH6.0 but affected the profile at pH7.4. More specifically, when 305LO15 was mixed with ECZ and hC5, peaks of the large ECZ/hC5/305LO15 immunocomplexes which were not detected at pH6.0 were detected at pH7.4 (see the peaks indicated by arrows in Figure 5, top), in addition to peaks of the small complexes (see peaks, '2Ag+Ab' and 'Ag+Ab', in Figure 5, top). Only the peaks of the small complexes were detected at pH6.0 (see the peaks of Figure 5, bottom, corresponding to '2Ag+Ab' and 'Ag+Ab' of Figure 5, top). This means that 305LO15 binds to ECZ/hC5 complex at pH7.4, but not at pH6.0, in accordance with the binding characteristics of 305LO15 (binding to hC5 at pH7.4 but not at pH6.0). From these results, it was suggested that simultaneous administration of 305LO15 and ECZ to a subject could result in the formation of large immunocomplexes in plasma.

### Example 7

Study BP39144, an adaptive phase I/II study, assessed safety, efficacy, pharmacokinetics (PK), and pharmacodynamics (PD) of 305LO15 in healthy volunteers and in patients with paroxysmal nocturnal hemoglobinuria (PNH).

Part 1 of Study BP39144 (a randomized, investigator/subject blinded, adaptive, placebo-controlled, parallel group study) evaluated the safety and tolerability of single doses of 305LO15 in healthy volunteers. At each dose-level/cohort, a total of 5 healthy volunteers were randomized to receive a single intravenous (IV; Cohorts 1 and 2) or subcutaneous (SC; Cohort 3) administration of 305LO15 or placebo. The study had an adaptive design, with an ongoing assessment of available safety, tolerability, PK, and PD data prior to the initiation of the next administration. Planned dose levels for cohorts 1, 2 and 3 were 75 mg IV, 150 mg IV, and 170 mg SC, respectively (Figure 4a). Based on preliminary review of the PK and PD data from Cohort 1, doses for Cohorts 2 and 3 were reduced to 125 mg IV (Cohort 2) and 100 mg SC (Cohort 3).

For IV infusion, blood samples were collected pre-dose, at the end of IV infusion (1 hour), 2, 6, 12, 24, 48, 72, 96, and 144 hours post-dose, and on days 14, 21, 28, 35, 42, 56, 84, and 91. For SC administration, blood samples were collected pre-dose, at 12, 24, 48, 72, 96 and 144 hours post-dose, and on days 14, 21, 28, 35, 42, 56, 84, and 91.

Preliminary safety results from Part 1 of Study BP39144 indicate that 305LO15 was well tolerated at all dose levels evaluated (75 and 125 mg IV and 100 mg SC). Available clinical safety information raised no significant safety concerns, and showed no evidence suggesting a pattern of adverse events or laboratory test abnormalities. There were no noteworthy findings or trends in vital signs or 12-lead electrocardiograms in any of the treatment groups.

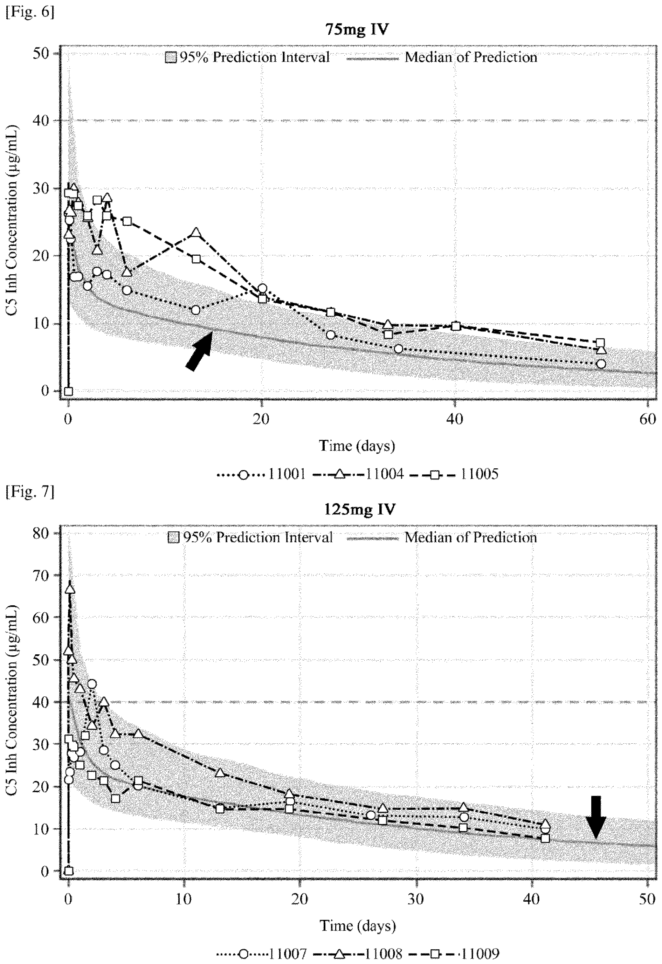

Preliminary PK results in serum and simulated concentration-time profiles for 305LO15 are presented in Figure 6, Figure 7, and Figure 8. Based on these data, 305LO15 PK in healthy subjects was in line with the initial predictions of human PK based on cynomolgus monkey PK data (Example 5). Following IV doses, exposure appeared to be dose proportional from 75 to 125 mg. The terminal half-life (t_{1/2}) for a typical patient of body weight 70 kg was estimated to be around 25 days. Following SC administrations, preliminary PK results showed that 305LO15 exposure peaked around day 7 and the bioavailability was around 90%. After absorption, the t_{1/2} was comparable with the t_{1/2} of the IV infusion.

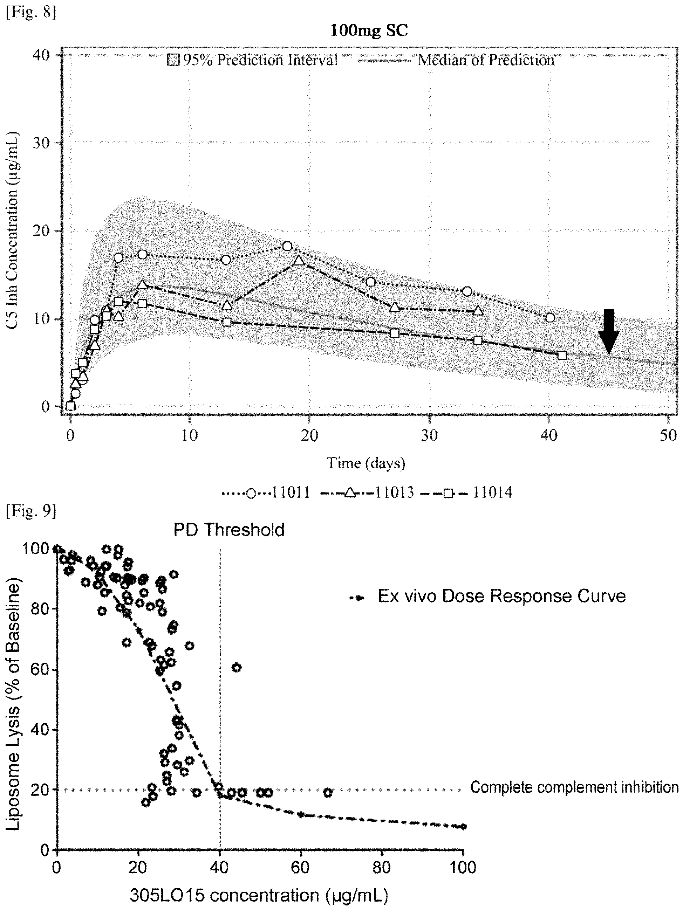

Preliminary assessment of the exposure-response relationship supported the initial prediction, i.e., approximately 40 micro g 305LO15 per mL blood is required to achieve complete complement inhibition (Figure 9).

### Example 8

Part 2 of Study BP39144 (an open-label, multiple-dose, global multicenter, intra-individual dose-escalation study) evaluated the safety and tolerability for a total duration of 5 months, and the pharmacodynamic effect of multiple doses of 305LO15 on complement activity in treatment naive PNH patients. In this study six PNH patients were enrolled. The enrolled patients had not been treated with any complement inhibitor or had previously been treated but stopped treatment due to lack of efficacy based on a single missense C5 heterozygous mutation, and showed increased serum LDH levels (>1.5 × ULN) at screening (ULN: upper limit of normal).

One patient (patient X) among six patients was a PNH patient and a candidate for treatment with complement inhibitors. Patient X received three single-ascending IV doses; a single infusion of 375 mg 305LO15 on day 1, followed by a single infusion of 500 mg 305LO15 on day 8, and further followed by a single infusion of 1000 mg 305LO15 on day 22. The first SC administration (170 mg) was initiated on day 36, followed by weekly (QW) SC injections (170 mg) of 305LO15 which will be continued for total treatment duration of 5 months.

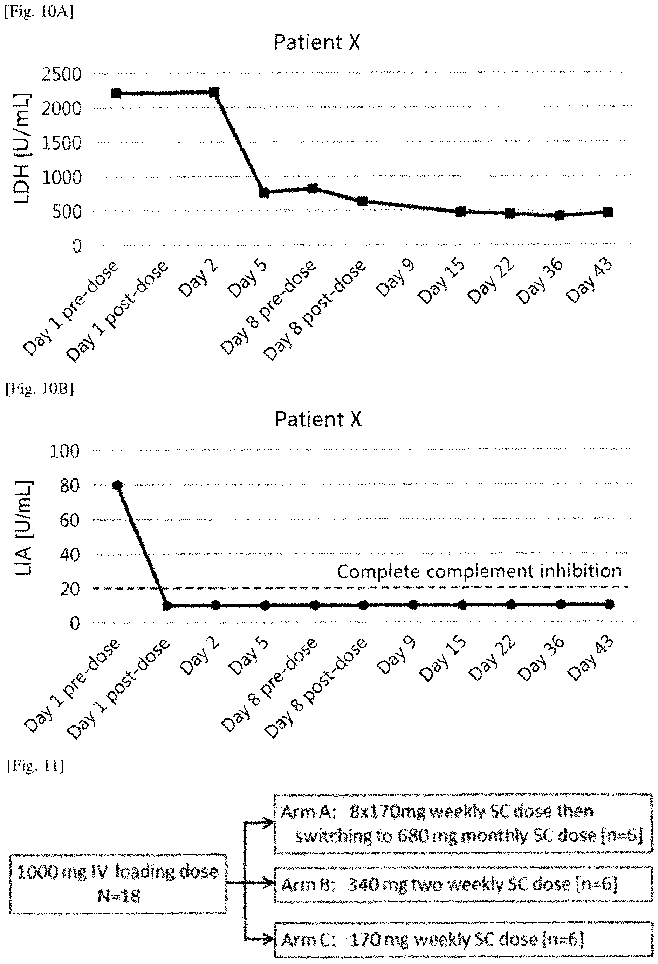

Preliminary pharmacodynamic results from Patient X are shown in Table 2 and Figure 10. LDH levels, as a pharmacodynamic marker of hemolysis, dropped to levels within the limits of the normal range by day 15, dropped further by day 36, and maintained at the normalized level on day 43 (Table 2 and Figure 10a). As shown in Table 2 and Figure 10b, results of liposome immunoassay (LIA) showed that complement activity was completely inhibited at the end of the infusion on the study-starting day, day 1. The dose of 375 mg 305LO15 on day 1 maintained complete complement inhibition until the next dose of 500 mg 305LO15 on day 8. The dose of 500 mg 305LO15 on day 8 maintained complete complement inhibition until the next dose of 1000 mg 305LO15 on day 22. The dose of 1000 mg 305LO15 on day 22 maintained complete complement inhibition until the next SC dose of 170 mg 305LO15 on day 36. The SC dose on day 36 maintained complete complement inhibition on day 43.

305LO15 was well tolerated with only mild abdominal pain unrelated to the treatment.

**[Table 2]**

| | LDH [U/mL] | LIA [U/mL] |
|---|---|---|
| Day 1 pre-dose | 2216 | 80 |
| Day 1 post-dose | Not collected | Complete inhibition |
| Day 2 | 2217 | Complete inhibition |
| Day 5 | 762 | Complete inhibition |
| Day 8 pre-dose | 824 | Complete inhibition |
| Day 8 post-dose | 634 | Complete inhibition |
| Day 9 | Hemolyzed | Complete inhibition |
| Day 15 | 474 | Complete inhibition |
| Day 22 | 455 | Complete inhibition |
| Day 36 | 414 | Complete inhibition |
| Day 43 | 461 | Complete inhibition |

### Example 9

Part 3 of Study BP39144 (an open-label, multiple-dose, global multicenter study) evaluated the safety and tolerability for a total duration of 5 months, and the pharmacodynamic effect of multiple doses of 305LO15 on complement activity in patients with PNH currently treated with eculizumab. In this study 18 PNH patients are to be enrolled. The enrolled patients had been treated continuously with eculizumab for at least 3 months preceding enrollment in the trial and the patients had to receive regular infusions of eculizumab.

One patient (patient Y) among 18 patients was a PNH patient treated with eculizumab, wherein eculizumab was finally administered 14 days before the first IV infusion of 305LO15. Patient Y received single IV loading dose; a single infusion of 1000 mg 305LO15 on day 1. The first SC administration (680 mg) was given on day 8, followed by every 4 week (Q4W) SC injections (680 mg) of 305LO15 which will be continued for total treatment duration of 5 months.

Preliminary pharmacodynamic results from Patient Y are shown in Table 3. LDH levels, as a pharmacodynamic marker of hemolysis, kept the baseline levels from the first dose to day 43 (Table 3). As shown in Table 3, results of liposome immunoassay (LIA) showed that complement activity was completely inhibited at the end of the infusion on the study-starting day, day 1. The dose of 1000 mg 305LO15 on day 1 maintained complete complement inhibition until the SC dose of 680 mg 305LO15 on day 8. The SC dose on day 8 maintained complete complement inhibition on day 43.

**[Table 3]**

| | LDH [U/mL] (103-229) | LIA [U/mL] |
|---|---|---|
| Day -1 | 328 | Complete inhibition |
| Day 1 pre-dose | 315 | Complete inhibition |
| Day 1 post-dose 10-12h | 421 | - |
| Day 2 | 273 | Complete inhibition |
| Day 8 pre-dose | 271 | Complete inhibition |
| Day 14 | 336 | Complete inhibition |
| Day 22 | 274 | Complete inhibition |
| Day 29 | - | Complete inhibition |
| Day 36 | 338 | Complete inhibition |
| Day 43 | 344 | Complete inhibition |

### Example 10

### Dose-Regimen Finding in Patients with PNH

This is an open-label, multiple-dose, global multicenter study in patients with PNH treated with eculizumab (see also 'Example 9'). Approximately eighteen adult male and female PNH patients who were treated with eculizumab were enrolled. The aim of Part 3 is to determine the optimal SC dose-regimen with low treatment burden for patients. Each studied dosing-regimen aims to achieve 305LO15 exposures that inhibit activation of the terminal complement pathway throughout the overall dosing period. The target exposure was defined based on available PK, PD, and efficacy data from Part 2. For all patients in Part 3 305LO15 dosing was initiated no later than two weeks after the patient's last dose of eculizumab. An IV loading dose was followed by repetitive SC maintenance dosing.

During Part 3, a part of the original SC dosing-regimen was altered for further safety improvement. Three different SC dosing-regimens (including weekly [QW], biweekly [Q2W], or monthly [Q1M] dosing) of 305LO15 are evaluated (see Figure 11). Patients assigned to Arm A receive SC dosing at 170 mg QW 8 times in the first phase. With the 9th SC dose (Study day 64) which is the first administration in the second phase, patients in Arm A start the 680 mg Q4W maintenance regimen. The IV loading dose is administered, for example, approximately 24 hours prior to the first SC dose for patients. In Part 3, patients receive treatment for a maximum of 5 months. In case a patient experiences signs and symptoms of his/her underlying disease such as breakthrough hemolysis which may be due to an acute event such as acute illness, trauma or surgery, etc., one or more additional IV doses of 305LO15 may be administered. Prior to this IV dose an unscheduled Biomarker PD sample should be drawn to evaluate the underlying cause of breakthrough hemolysis.

Preliminary pharmacodynamic results from Patients Z1 and Z2 in Arm A are shown in Table 4. LDH levels, as a pharmacodynamic marker of hemolysis, kept the baseline levels from the first dose to day 106 (Table 4). As shown in Table 4, results of liposome immunoassay (LIA) showed that complement activity was completely inhibited at the end of the infusion on the study-starting day, day 1. The dose of 1000 mg 305LO15 on day 1 maintained complete complement inhibition until the first SC dose of 170 mg 305LO15 on day 8. Since the 9th SC dose (Study day 64), those patients have received the 680 mg Q4W maintenance regimen. The complete complement inhibition (LIA [U/mL] < 10) in Patient Z2 has been maintained during SC doses (day 64 to day 106). Complement inhibition in Patient Z1 has been completely or sufficiently inhibited during SC doses (day 64 to day 106). The Arm A regimen has been well tolerated with only mild adverse events (shoulder paresthesia in patient Z1, common cold in patient Z2) unrelated to the treatment.

**[Table 4]**

| | Patient Z1 | | Patient Z2 | |
|---|---|---|---|---|
| | LDH [U/mL] | LIA [U/mL] | LDH [U/mL] | LIA [U/mL] |
| Day -1 | 433 | < 10 | 305 | < 10 |
| Day 1 pre-dose | 408 | < 10 | 313 | 12 |
| Day 1 post-dose | - | < 10 | - | < 10 |
| Day 2 | 367 | < 10 | 253 | < 10 |
| Day 8 pre-dose | 370 | < 10 | 248 | < 10 |
| Day 15 | 365 | < 10 | 293 | < 10 |
| Day 22 | 431 | < 10 | 313 | < 10 |
| Day 29 | 408 | 11 | 316 | < 10 |
| Day 36 | 428 | < 10 | 307 | < 10 |
| Day 43 | 473 | < 10 | 333 | < 10 |
| Day 64 | 435 | < 10 | 343 | < 10 |
| Day 78 | 445 | 11 | 329 | < 10 |
| Day 92 | 419 | 11 | 272 | < 10 |
| Day 106 | 448 | < 10 | 305 | < 10 |

### Example 11

### Open Label Extension, OLE, in Patients with PNH

This is an OLE for patients who participated in Part 3 and who derived benefit from treatment with 305LO15. The number of enrolled patients does not exceed the numbers enrolled in the Part 3 study of 'Example 10'. Patients transitioning from the Part 3 study initially remain on the same SC dose regimen as they were receiving in Part 3. Depending on emerging safety, PK, and PD data from the Part 3 study, the SC dosing regimen for patients enrolled in the OLE is adapted accordingly. Treatment duration is up to a maximum of two years from entry into OLE.

Furthermore, the invention relates to the following items:

| | |
|---|---|
| [Item 1] | A pharmaceutical composition for use in a method of treatment or prevention of a C5-related disease, which is formulated for subcutaneous injection, and comprises an anti-C5 antibody, wherein the composition is subcutaneously administered in two phases, wherein in both phases there is an interval between every two subcutaneous administrations, |
| | wherein each phase comprises at least one interval, and wherein in the first phase |
| | i) the at least one interval is shorter than the at least one interval in the second phase, and |
| | ii) the dose of the antibody per administration is lower than or the same as the antibody dose per administration in the second phase. |
| [Item 2] | The pharmaceutical composition for use of item 1, wherein the at least one interval in the first phase is 1 day to 2 months. |
| [Item 3] | The pharmaceutical composition for use of item 1 or 2, wherein the at least one interval in the first phase is 5 days to 14 days. |
| [Item 4] | The pharmaceutical composition for use of any one of items 1 to 3, wherein the at least one interval in the second phase is 2 days to 6 months. |
| [Item 5] | The pharmaceutical composition for use of any one of items 1 to 4, wherein said at least one interval in the second phase is 15 days to 3 months. |
| [Item 6] | The pharmaceutical composition for use of any one of items 1 to 5, wherein the dose of the antibody in the subcutaneous administration of the first phase is 50 mg to 350 mg. |
| [Item 7] | The pharmaceutical composition for use of any one of items 1 to 6, wherein the dose of the antibody of the subcutaneous administration of the first phase is 150 mg to 200 mg and lower than the dose of subcutaneous administration of the second phase, preferably wherein the antibody dose in the first phase is 170mg. |
| [Item 8] | The pharmaceutical composition for use of any one of items 1 to 6, wherein the dose of the antibody of the subcutaneous administration of the first phase is 300 mg to 350 mg and the same as the antibody dose of the subcutaneous administration of the second phase. |
| [Item 9] | The pharmaceutical composition for use of any one of items 1 to 8, wherein number of subcutaneous administrations in the first phase is 1 |
| | to 12. |
| [Item 10] | The pharmaceutical composition for use of any one of items 1 to 9, number of subcutaneous administrations in the first phase is 5 to 10, preferably wherein the number is 8. |
| [Item 11] | The pharmaceutical composition for use of any one of items 1 to 10, wherein the dose of the antibody per administration in the second phase is 350 mg to 1,000 mg, or 650 mg to 700 mg. |
| [Item 12] | The pharmaceutical composition for use of any one of items 1 to 7 and 9 to 11, wherein the anti-C5 antibody dose per administration in the first phase is three to five folds lower than the anti-C5 antibody dose per administration in the second phase. |
| [Item 13] | The pharmaceutical composition for use of any one of items 1 to 12, wherein a pharmaceutical composition formulated for intravenous administration and comprising an anti-C5 antibody is intravenously administrated before the first subcutaneous administration of the first phase. |
| [Item 14] | The pharmaceutical composition for use of item 13, wherein the first subcutaneous administration of the first phase is administrated 0 days to 1 month after the final administration of the intravenously administrated pharmaceutical composition. |
| [Item 15] | The pharmaceutical composition for use of item 13 or 14, wherein the antibody dose of said intravenous administration is 100 to 2,000 mg. |
| [Item 16] | The pharmaceutical composition for use of any one of items 1 to 15, wherein the C5-related disease is any one selected from a group consisting of rheumatoid arthritis (RA); lupus nephritis; ischemia-reperfusion injury; paroxysmal nocturnal hemoglobinuria (PNH); atypical hemolytic uremic syndrome (aHUS); dense deposit disease (DDD); macular degeneration; hemolysis, elevated liver enzymes, and low platelets (HELLP) syndrome; thrombotic thrombocytopenic purpura (TTP); spontaneous fetal loss; Pauci-immune vasculitis; epidermolysis bullosa; recurrent fetal loss; multiple sclerosis (MS); traumatic brain injury; and injury resulting from myocardial infarction, cardiopulmonary bypass or hemodialysis. |
| [Item 17] | A method for treating or preventing a C5-related disease, wherein the method comprises subcutaneously administering to a subject a pharmaceutical composition, which is formulated for subcutaneous injection and comprises an anti-C5 antibody, wherein the composition is subcutaneously administered in two phases, |
| | wherein in both phases there is an interval between every two subcutaneous administrations, wherein each phase comprises at least one interval, and wherein in the first phase |
| | i) the at least one interval is shorter than the at least one interval in the second phase, and |
| | ii) the dose of the antibody per administration is lower than or the same as the antibody dose per administration in the second phase. |
| [Item 18] | Use of an anti-C5 antibody in the manufacture of a pharmaceutical composition for treating or preventing a C5-related disease, wherein the composition is formulated for subcutaneous injection, comprises the anti-C5 antibody, and wherein the composition is subcutaneously administered in two phases, |
| | wherein in both phases there is an interval between every two subcutaneous administrations, wherein each phase comprises at least one interval, and wherein in the first phase |
| | i) the at least one interval is shorter than the at least one interval in the second phase, and |
| | ii) the dose of the antibody per administration is lower than or the same as the antibody dose per administration in the second phase. |
| [Item 19] | A product for treating or preventing a C5-related disease, comprising (a) a container; (b) a pharmaceutical composition in the container, wherein the pharmaceutical composition is formulated for subcutaneous injection and comprises an anti-C5 antibody; and (c) a document instructing that the pharmaceutical composition is subcutaneously administered in two phases, |
| | wherein in both phases there is an interval between every two subcutaneous administrations, wherein each phase comprises at least one interval, and wherein in the first phase |
| | i) the at least one interval is shorter than the at least one interval in the second phase, and |
| | ii) the dose of the antibody per administration is lower than or the same as the antibody dose per administration in the second phase. |

## Claims

1. A pharmaceutical composition formulated for subcutaneous injection comprising an anti-C5 antibody for use in a method of treatment or prevention of a C5-related disease,
wherein the composition subcutaneously administered in two phases after a pharmaceutical composition formulated for intravenous injection comprising an anti-C5 antibody is administered,
wherein in both phases of the subcutaneous administration there is an interval between every two subcutaneous administrations, wherein each phase comprises at least one interval, wherein
(i) in the first phase the at least one interval is shorter than the at least one interval in the second phase, and
(ii) the dose of the antibody per administration in the first phase is lower than or the same as the antibody dose per administration in the second phase,
wherein the antibody dose of the intravenous administration is 1000 to 2,000 mg,
wherein the first subcutaneous administration of the first phase is administered 0 to 8 days after the final administration of the intravenously administered pharmaceutical composition, wherein the dose of the antibody in the subcutaneous administration of the first phase is 170 mg or 340 mg, and
wherein the pharmaceutical composition is administered in the first phase once every week (weekly).

2. The pharmaceutical composition for use of claim 1, wherein the pharmaceutical composition for subcutaneous administration is administered in the second phase at an interval of 15 day to 3 months.

3. The pharmaceutical composition for use of claim 1 or 2, wherein the dose of the antibody in the subcutaneous administration of the first phase is three to five folds lower than the dose of the antibody in the subcutaneous administration of the second phase.

4. The pharmaceutical composition for use of claims 1 to 3, comprising the VH sequence of SEQ ID NO: 2 and the VL sequence of SEQ ID NO: 6.

5. The pharmaceutical composition for use of claims 1 to 4, wherein the C5-related disease is selected from the group consisting of rheumatoid arthritis (RA); lupus nephritis; ischemia-reperfusion injury; paroxysmal nocturnal hemoglobinuria (PNH), atypical hemolytic uremic syndrome (aHUS), dense deposit disease (DDD); macular degeneration; hemolysis, elevated liver enzymes, and low platelets (HELLP) syndrome; thrombotic thrombocytopenic purpura (TTP); spontaneous fetal loss; Pauci-immune vasculitis; epidermolysis bullosa; recurrent fetal loss; multiple sclerosis (MS); traumatic brain injury; injury resulting from myocardial infarction, cardiopulmonary bypass or hemodialysis; refractory generalized myasthenia gravis (gMG), and neuromyelitis optica (NMO).
